# EUROPEAN PATENT SPECIFICATION

(11) **EP 2 570 813 B1**
(45) Date of publication and mention of the grant of the patent: **17.06.2020**
(21) Application number: 11780257.9
(22) Date of filing: 13.05.2011
(51) Int. Cl.: G01N 33/574

(54) **METHOD FOR THE DIAGNOSIS/PROGNOSIS OF COLORECTAL CANCER**
VERFAHREN ZUR DIAGNOSE/PROGNOSE VON DARMKREBS
MÉTHODE DE DIAGNOSTIC/PRONOSTIC DU CANCER COLORECTAL

(30) Priority: 13.05.2010 ES 201030708
(43) Date of publication of application: 20.03.2013
(73) Proprietor: Consejo Superior De Investigaciones Científicas (CSIC), 28006 Madrid (ES)
(72) Inventor: CASAL ÁLVAREZ, José Ignacio, E-28040 Madrid (ES); BARDERAS MANCHADO, Rodrigo, E-28040 Madrid (ES); BABEL, Ingrid Henriette Suzanne, E-28040 Madrid (ES)
(74) Representative: ABG Intellectual Property Law, S.L.
(86) International application number: PCT/ES2011/070346
(87) International publication number: WO 2011/141612

(56) References cited:
- WO-A1-2009/008496
- WO-A2-2009/040782
- BABEL INGRID ET AL: "Identification of Tumor-associated Autoantigens for the Diagnosis of Colorectal Cancer in Serum Using High Density Protein Microarrays", MOLECULAR & CELLULAR PROTEOMICS, AMERICAN SOCIETY FOR BIOCHEMISTRY AND MOLECULAR BIOLOGY, vol. 8, no. 10, 1 October 2009 (2009-10-01), pages 2382-2395, XP009137622, ISSN: 1535-9476, DOI: 10.1074/MCP.M800596-MCP200 [retrieved on 2009-08-28]
- RAN YULIANG ET AL: "Profiling tumor-associated autoantibodies for the detection of colon cancer", CLINICAL CANCER RESEARCH, THE AMERICAN ASSOCIATION FOR CANCER RESEARCH, US, vol. 14, no. 9, 1 May 2008 (2008-05-01), pages 2696-2700, XP002596849, ISSN: 1078-0432, DOI: 10.1158/1078-0432.CCR-07-2021
- SOMERS VEERLE A ET AL: "A panel of candidate tumor antigens in colorectal cancer revealed by the serological selection of a phage displayed cDNA expression library", THE JOURNAL OF IMMUNOLOGY, THE AMERICAN ASSOCIATION OF IMMUNOLOGISTS, US, vol. 169, no. 5, 1 September 2002 (2002-09-01), pages 2772-2780, XP002517011, ISSN: 0022-1767
- KIJANKA G ET AL: "Human IgG antibody profiles differentiate between symptomatic patients with and without colorectal cancer", GUT, vol. 59, no. 1, January 2010 (2010-01), pages 69-78, XP009173900, ISSN: 0017-5749
- REIPING TANG ET AL: "Humoral response to p53 in human colorectal tumors: A prospective study of 1,209 patients", INTERNATIONAL JOURNAL OF CANCER, vol. 94, no. 6, 15 December 2001 (2001-12-15), pages 859-863, XP055085702, ISSN: 0020-7136, DOI: 10.1002/ijc.1541
- CHUNG-CHUAN CHAN ET AL: "Multiple serological biomarkers for colorectal cancer detection", INTERNATIONAL JOURNAL OF CANCER, 1 January 2010 (2010-01-01), pages NA-NA, XP055086360, ISSN: 0020-7136, DOI: 10.1002/ijc.24912
- ROB C. ROOVERS ET AL: "Evidence for a bias toward intracellular antigens in the local humoral anti-tumor immune response of a colorectal cancer patient revealed by phage display", INTERNATIONAL JOURNAL OF CANCER, vol. 93, no. 6, 15 September 2001 (2001-09-15), pages 832-840, XP055085819, ISSN: 0020-7136, DOI: 10.1002/ijc.1382
- JAN FRIEDERICHS ET AL: "Gene expression profiles of different clinical stages of colorectal carcinoma: toward a molecular genetic understanding of tumor progression", INTERNATIONAL JOURNAL OF COLORECTAL DISEASE ; CLINICAL AND MOLECULAR GASTROENTEROLOGY AND SURGERY, SPRINGER, BERLIN, DE, vol. 20, no. 5, 1 September 2005 (2005-09-01), pages 391-402, XP019340240, ISSN: 1432-1262, DOI: 10.1007/S00384-004-0722-1
- MADOZ-GURPIDE J ET AL: "Proteomics-based validation of genomic data: applications in colorectal cancer diagnosis", MOLECULAR & CELLULAR PROTEOMICS, AMERICAN SOCIETY FOR BIOCHEMISTRY AND MOLECULAR BIOLOGY, vol. 5, no. 8, 29 May 2006 (2006-05-29), pages 1471-1483, XP002463510, ISSN: 1535-9476, DOI: 10.1074/MCP.M600048-MCP200
- CARDOSO ET AL: "Expression and genomic profiling of colorectal cancer", BBA - REVIEWS ON CANCER, ELSEVIER SCIENCE BV, AMSTERDAM, NL, vol. 1775, no. 1, 13 December 2006 (2006-12-13), pages 103-137, XP005802353, ISSN: 0304-419X, DOI: 10.1016/J.BBCAN.2006.08.004
- KALNINA Z ET AL: "Evaluation of T7 and lambda phage display systems for survey of autoantibody profiles in cancer patients", JOURNAL OF IMMUNOLOGICAL METHODS, ELSEVIER SCIENCE PUBLISHERS B.V.,AMSTERDAM, NL, vol. 334, no. 1-2, 20 May 2008 (2008-05-20), pages 37-50, XP022614832, ISSN: 0022-1759, DOI: 10.1016/J.JIM.2008.01.022 [retrieved on 2008-02-21]
- W. CHANG ET AL: "Development of Autoantibody Signatures as Biomarkers for Early Detection of Colorectal Carcinoma", CLINICAL CANCER RESEARCH, vol. 17, no. 17, 19 July 2011 (2011-07-19) , pages 5715-5724, XP055085848, ISSN: 1078-0432, DOI: 10.1158/1078-0432.CCR-11-0199
- KLEIN-SCORY, S. ET AL.: 'Immunoscreening of the extracellular proteome of colorectal cancer cells.' BMC CANCER vol. 10, 25 February 2010, ISSN 1471-2407 page 70
- BABEL, I. ET AL.: 'Identification of tumor-associated autoantigens for the diagnosis of colorectal cancer in serum using high density protein microarrays.' MOLECULAR & CELLULAR PROTEOMICS vol. 8, no. 10, October 2009, ISSN 1535-9476 pages 2382 - 2395
- CHAN, C.C. ET AL.: 'Multiple serological biomarkers for colorectal cancer detection.' INTERNATIONAL JOURNAL OF CANCER vol. 126, no. 7, April 2010, ISSN 0020-7136 pages 1683 - 1690
- BARDERAS, R. ET AL.: 'Colorectal cancer proteomics, molecular characterization and biomarker discovery' PROTEOMICS CLINICAL APPLICATIONS vol. 4, no. 2, February 2010, ISSN 1862-8346 pages 159 - 178
- TAN, H.T. ET AL.: 'Serum autoantibodies as biomarkers for early cancer detection.' FEBS JOURNAL vol. 276, no. 23, 2009, ISSN 1742-4658 pages 6880 - 6904
- BABEL, I. ET AL.: 'Identification of MSTl/STK4 and SULF1 proteins as autoantibody targets for the diagnosis of colorectal cancer by using phage microarrays' MOLECULAR & CELLULAR PROTEOMICS vol. 10, no. 3, March 2011, ISSN 1535-9476 page M110.001784

### LISTA DE SECUENCIAS

<110> CONSEJO SUPERIOR DE INVESTIGACIONES CIENTÍFICAS
<120> MÉTODO DE DIAGNÓSTICO/PRONÓSTICO DEL CANCER COLORRECTAL
<130> P6917PC00
<150> ES201030708
   <151> 2010-05-13
<160> 14
<170> PatentIn version 3.5
<210> 1
   <211> 7
   <212> PRT
   <213> Homo sapiens
<400> 1
   Ser Arg Ala Thr Met Pro Gly
   1               5
<210> 2
   <211> 14
   <212> PRT
   <213> Homo sapiens
<400> 2
   Ser Leu Gly Gln Arg Ser Gln Lys Leu Ala Ala Ala Leu Glu
   1               5                   10
<210> 3
   <211> 8
   <212> PRT
   <213> Homo sapiens
<400> 3
   Tyr Asn Ser Lys Arg Gln Pro Asn
   1               5
<210> 4
   <211> 8
   <212> PRT
   <213> Homo sapiens
<400> 4
   Glu Asn Lys Thr Arg Lys Ile Met
   1               5
<210> 5
   <211> 10
   <212> PRT
   <213> Homo sapiens
<400> 5
   Arg Asp Arg Ala Thr Ala Ile Gln Pro Gly
   1               5                   10
<210> 6
   <211> 5
   <212> PRT
   <213> Homo sapiens
<400> 6
   Ser Ser Lys Ser Pro
   1               5
<210> 7
   <211> 1606
   <212> PRT
   <213> Homo sapiens
<400> 7
   Met Val Val Phe Ile Asn Ala Lys Ile Lys Ser Leu Ile Lys Leu Phe
   1               5                   10                  15
   Lys Lys Lys Thr Val Ser Asn Leu Asp Glu Glu Ser Arg Trp Thr Val
               20                  25                  30
   His Tyr Thr Ala Pro Trp His Gln Gln Glu Asn Val Phe Leu Pro Thr
           35                  40                  45
   Thr Arg Pro Pro Cys Val Glu Asp Leu His Arg Gln Ala Lys Leu Asn
       50                  55                  60
   Leu Lys Ser Val Leu Arg Glu Cys Asp Lys Leu Arg His Asp Gly Tyr
   65                  70                  75                  80
   Arg Ser Ser Gln Tyr Tyr Ser Gln Gly Pro Thr Phe Ala Ala Asn Ala
                   85                  90                  95
   Ser Pro Phe Cys Asp Asp Tyr Gln Asp Glu Asp Glu Glu Thr Asp Gln
               100                 105                 110
   Lys Cys Ser Leu Ser Ser Ser Glu Glu Glu Arg Phe Ile Ser Ile Arg
           115                 120                 125
   Arg Pro Lys Thr Pro Ala Ser Ser Asp Phe Ser Asp Leu Asn Thr Gln
       130                 135                 140
   Thr Asn Trp Thr Lys Ser Leu Pro Leu Pro Thr Pro Glu Glu Lys Met
   145                 150                 155                 160
   Arg Gln Gln Ala Gln Thr Val Gln Ala Asp Val Val Pro Ile Asn Ile
                   165                 170                 175
   Thr Gly Glu Asn Phe Asp Arg Gln Ala Ser Leu Arg Arg Ser Leu Ile
   180 185 190
   Tyr Thr Asp Thr Leu Val Arg Arg Pro Lys Lys Val Lys Arg Arg Lys
   195 200 205
   Thr Ile Thr Gly Val Pro Asp Asn Ile Gln Lys Glu Leu Ala Ser Gly
   210 215 220
   Thr Gly Gln Asp Asp Ala Asp Gly His Ser Val Tyr Thr Pro Asp His
   225 230 235 240
   Tyr Ser Thr Leu Gly Arg Phe Asn Ser Cys Arg Ser Ala Gly Gln Arg
   245 250 255
   Ser Glu Thr Arg Asp Ser Ser Cys Gln Thr Glu Asp Val Lys Val Val
   260 265 270
   Pro Pro Ser Met Arg Arg Ile Arg Ala Gln Lys Gly Gln Gly Ile Ala
   275 280 285
   Ala Gln Met Gly His Phe Ser Gly Ser Ser Gly Asn Met Ser Val Leu
   290 295 300
   Ser Asp Ser Ala Gly Ile Val Phe Pro Ser Arg Leu Asp Ser Asp Ala
   305 310 315 320
   Gly Phe His Ser Leu Pro Arg Ser Gly Ala Arg Ala Asn Ile Gln Ser
   325 330 335
   Leu Glu Pro Arg Leu Gly Ala Leu Gly Pro Ala Gly Asp Met Asn Gly
   340 345 350
   Thr Phe Leu Tyr Gln Arg Gly His Pro Gln Ala Asp Glu Asn Leu Gly
   355 360 365
   His Leu Gly Gly Ala Ser Gly Thr Gly Thr Leu Leu Arg Pro Lys Ser
   370 375 380
   Gln Glu Leu Arg His Phe Glu Ser Glu Asn Ile Met Ser Pro Ala Cys
   385 390 395 400
   Val Val Ser Pro His Ala Thr Tyr Ser Thr Ser Ile Ile Pro Asn Ala
   405 410 415
   Thr Leu Ser Ser Ser Ser Glu Val Ile Ala Ile Pro Thr Ala Gln Ser
   420 425 430
   Ala Gly Gln Arg Glu Ser Lys Ser Ser Gly Ser Ser His Ala Arg Ile
   435 440 445
   Lys Ser Arg Asp His Leu Ile Ser Arg His Ala Val Lys Gly Asp Pro
   450 455 460
   Gln Ser Pro Gly Arg His Trp Asn Glu Gly His Ala Thr Ile Leu Ser
   465 470 475 480
   Gln Asp Leu Asp Pro His Ser Pro Gly Glu Pro Ala Leu Leu Ser Leu
   485 490 495
   Cys Asp Ser Ala Val Pro Leu Asn Ala Pro Ala Asn Arg Glu Asn Gly
   500 505 510
   Ser Gln Ala Met Pro Tyr Asn Cys Arg Asn Asn Leu Ala Phe Pro Ala
   515 520 525
   His Pro Gln Asp Val Asp Gly Lys Ser Glu Ser Ser Tyr Ser Gly Gly
   530 535 540
   Gly Gly His Ser Ser Ser Glu Pro Trp Glu Tyr Lys Ser Ser Gly Asn
   545 550 555 560
   Gly Arg Ala Ser Pro Leu Lys Pro His Leu Ala Thr Pro Gly Tyr Ser
   565 570 575
   Thr Pro Thr Ser Asn Met Ser Ser Cys Ser Leu Asp Gln Thr Ser Asn
   580 585 590
   Lys Glu Asp Ala Gly Ser Leu Tyr Ser Glu Asp His Asp Gly Tyr Cys
   595 600 605
   Ala Ser Val His Thr Asp Ser Gly His Gly Ser Gly Asn Leu Cys Asn
   610 615 620
   Ser Ser Asp Gly Phe Gly Asn Pro Arg His Ser Val Ile Asn Val Phe
   625 630 635 640
   Val Gly Arg Ala Gln Lys Asn Gln Gly Asp Arg Ser Asn Tyr Gln Asp
   645 650 655
   Lys Ser Leu Ser Arg Asn Ile Ser Leu Lys Lys Ala Lys Lys Pro Pro
   660 665 670
   Leu Pro Pro Ser Arg Thr Asp Ser Leu Arg Arg Ile Pro Lys Lys Ser
   675 680 685
   Ser Gln Cys Asn Gly Gln Val Leu Asn Glu Ser Leu Ile Ala Thr Leu
       690                 695                 700
   Gln His Ser Leu Gln Leu Ser Leu Pro Gly Lys Ser Gly Ser Ser Pro
   705                 710                 715                 720
   Ser Gln Ser Pro Cys Ser Asp Leu Glu Glu Pro Trp Leu Pro Arg Ser
                   725                 730                 735
   Arg Ser Gln Ser Thr Val Ser Ala Gly Ser Ser Met Thr Ser Ala Thr
               740                 745                 750
   Thr Pro Asn Val Tyr Ser Leu Cys Gly Ala Thr Pro Ser Gln Ser Asp
           755                 760                 765
   Thr Ser Ser Val Lys Ser Glu Tyr Thr Asp Pro Trp Gly Tyr Tyr Ile
       770                 775                 780
   Asp Tyr Thr Gly Met Gln Glu Asp Pro Gly Asn Pro Ala Gly Gly Cys
   785                 790                 795                 800
   Ser Thr Ser Ser Gly Val Pro Thr Gly Asn Gly Pro Val Arg His Val
                   805                 810                 815
   Gln Glu Gly Ser Arg Ala Thr Met Pro Gln Val Pro Gly Gly Ser Val
               820                 825                 830
   Lys Pro Lys Ile Met Ser Pro Glu Lys Ser His Arg Val Ile Ser Pro
           835                 840                 845
   Ser Ser Gly Tyr Ser Ser Gln Ser Asn Thr Pro Thr Ala Leu Thr Pro
       850                 855                 860
   Val Pro Val Phe Leu Lys Ser Val Ser Pro Ala Asn Gly Lys Gly Lys
   865                 870                 875                 880
   Pro Lys Pro Lys Val Pro Glu Arg Lys Ser Ser Leu Ile Ser Ser Val
                   885                 890                 895
   Ser Ile Ser Ser Ser Ser Thr Ser Leu Ser Ser Ser Thr Ser Thr Glu
               900                 905                 910
   Gly Ser Gly Thr Met Lys Lys Leu Asp Pro Ala Val Gly Ser Pro Pro
           915                 920                 925
   Ala Pro Pro Pro Pro Pro Val Pro Ser Pro Pro Phe Pro Cys Pro Ala
       930                 935                 940
   Asp Arg Ser Pro Phe Leu Pro Pro Pro Pro Pro Val Thr Asp Cys Ser
   945 950 955 960
   Gln Gly Ser Pro Leu Pro His Ser Pro Val Phe Pro Pro Pro Pro Pro
   965 970 975
   Glu Ala Leu Ile Pro Phe Cys Ser Pro Pro Asp Trp Cys Leu Ser Pro
   980 985 990
   Pro Arg Pro Ala Leu Ser Pro Ile Leu Pro Asp Ser Pro Val Ser Leu
   995 1000 1005
   Pro Leu Pro Pro Pro Leu Leu Pro Ser Ser Glu Pro Pro Pro Ala
   1010 1015 1020
   Pro Pro Leu Asp Pro Lys Phe Met Lys Asp Thr Arg Pro Pro Phe
   1025 1030 1035
   Thr Asn Ser Gly Gln Pro Glu Ser Ser Arg Gly Ser Leu Arg Pro
   1040 1045 1050
   Pro Ser Thr Lys Glu Glu Thr Ser Arg Pro Pro Met Pro Leu Ile
   1055 1060 1065
   Thr Thr Glu Ala Leu Gln Met Val Gln Leu Arg Pro Val Arg Lys
   1070 1075 1080
   Asn Ser Gly Ala Glu Ala Ala Gln Leu Ser Glu Arg Thr Ala Gln
   1085 1090 1095
   Glu Gln Arg Thr Pro Val Ala Pro Gln Tyr His Leu Lys Pro Ser
   1100 1105 1110
   Ala Phe Leu Lys Ser Arg Asn Ser Thr Asn Glu Met Glu Ser Glu
   1115 1120 1125
   Ser Gln Pro Ala Ser Val Thr Ser Ser Leu Pro Thr Pro Ala Lys
   1130 1135 1140
   Ser Ser Ser Gln Gly Asp His Gly Ser Ala Ala Glu Arg Gly Gly
   1145 1150 1155
   Pro Val Ser Arg Ser Pro Gly Ala Pro Ser Ala Gly Glu Ala Glu
   1160 1165 1170
   Ala Arg Pro Ser Pro Ser Thr Thr Pro Leu Pro Asp Ser Ser Pro
   1175 1180 1185
   Ser Arg Lys Pro Pro Pro Ile Ser Lys Lys Pro Lys Leu Phe Leu
   1190 1195 1200
   Val Val  Pro Pro Pro Gln Lys  Asp Phe Ala Val Glu  Pro Ala Glu
       1205                 1210                 1215
   Asn Val  Ser Glu Ala Leu Arg  Ala Val Pro Ser Pro  Thr Thr Gly
       1220                 1225                 1230
   Glu Glu  Gly Ser Val His Ser  Arg Glu Ala Lys Glu  Ser Ser Ala
       1235                 1240                 1245
   Ala Gln  Ala Gly Ser His Ala  Thr His Pro Gly Thr  Ser Val Leu
       1250                 1255                 1260
   Glu Gly  Gly Ala Ala Gly Ser  Met Ser Pro Ser Arg  Val Glu Ala
       1265                 1270                 1275
   Asn Val  Pro Met Val Gln Pro  Asp Val Ser Pro Ala  Pro Lys Gln
       1280                 1285                 1290
   Glu Glu  Pro Ala Glu Asn Ser  Ala Asp Thr Gly Gly  Asp Gly Glu
       1295                 1300                 1305
   Ser Cys  Leu Ser Gln Gln Asp  Gly Ala Ala Gly Val  Pro Glu Thr
       1310                 1315                 1320
   Asn Ala  Ala Gly Ser Ser Ser  Glu Ala Cys Asp Phe  Leu Lys Glu
       1325                 1330                 1335
   Asp Gly  Asn Asp Glu Val Met  Thr Pro Ser Arg Pro  Arg Thr Thr
       1340                 1345                 1350
   Glu Asp  Leu Phe Ala Ala Ile  His Arg Ser Lys Arg  Lys Val Leu
       1355                 1360                 1365
   Gly Arg  Arg Asp Ser Asp Asp  Asp His Ser Arg Asn  His Ser Pro
       1370                 1375                 1380
   Ser Pro  Pro Val Thr Pro Thr  Gly Ala Ala Pro Ser  Leu Ala Ser
       1385                 1390                 1395
   Pro Lys  Gln Val Gly Ser Ile  Gln Arg Ser Ile Arg  Lys Ser Ser
       1400                 1405                 1410
   Thr Ser  Ser Asp Asn Phe Lys  Ala Leu Leu Leu Lys  Lys Gly Ser
       1415                 1420                 1425
   Arg Ser  Asp Thr Ser Ala Arg  Met Ser Ala Ala Glu  Met Leu Lys
       1430                 1435                 1440
   Asn Thr Asp Pro Arg Phe Gln Arg Ser Arg Ser Glu Pro Ser Pro
       1445                1450                1455
   Asp Ala Pro Glu Ser Pro Ser Ser Cys Ser Pro Ser Lys Asn Arg
       1460                1465                1470
   Arg Ala Gln Glu Glu Trp Ala Lys Asn Glu Gly Leu Met Pro Arg
       1475                1480                1485
   Ser Leu Ser Phe Ser Gly Pro Arg Tyr Gly Arg Ser Arg Thr Pro
       1490                1495                1500
   Pro Ser Ala Ala Ser Ser Arg Tyr Ser Met Arg Asn Arg Ile Gln
       1505                1510                1515
   Ser Ser Pro Met Thr Val Ile Ser Glu Gly Glu Gly Glu Ala Val
       1520                1525                1530
   Glu Pro Val Asp Ser Ile Ala Arg Gly Ala Leu Gly Ala Ala Glu
       1535                1540                1545
   Gly Cys Ser Leu Asp Gly Leu Ala Arg Glu Glu Met Asp Glu Gly
       1550                1555                1560
   Gly Leu Leu Cys Gly Glu Gly Pro Ala Ala Ser Leu Gln Pro Gln
       1565                1570                1575
   Ala Pro Gly Pro Val Asp Gly Thr Ala Ser Ala Glu Gly Arg Glu
       1580                1585                1590
   Pro Ser Pro Gln Cys Gly Gly Ser Leu Ser Glu Glu Ser
       1595                1600                1605
<210> 8
   <211> 69
   <212> PRT
   <213> Homo sapiens
<400> 8
   Ala Met Val Pro Cys Asp Asn Val Ser Ser Cys Pro Ser Ser Asp Thr
   1               5                   10                  15
   Cys Cys Gln Leu Thr Ser Gly Glu Trp Gly Cys Cys Pro Ile Pro Glu
               20                  25                  30
   Ala Val Cys Cys Ser Asp His Gln His Cys Cys Pro Gln Gly Tyr Thr
           35                  40                  45
   Cys Val Ala Glu Gly Gln Cys Gln Lys Leu Ala Ala Ala Leu Glu His
       50                  55                  60
   His His His His His
   65
<210> 9
   <211> 487
   <212> PRT
   <213> Homo sapiens
<400> 9
<210> 10
   <211> 882
   <212> PRT
   <213> Homo sapiens
<400> 10
   Met Lys Tyr Ser Cys Cys Ala Leu Val Leu Ala Val Leu Gly Thr Glu
   1               5                   10                  15
   Leu Leu Gly Ser Leu Cys Ser Thr Val Arg Ser Pro Arg Phe Arg Gly
               20                  25                  30
   Arg Ile Gln Gln Glu Arg Lys Asn Ile Arg Pro Asn Ile Ile Leu Val
           35                  40                  45
   Leu Thr Asp Asp Gln Asp Val Glu Leu Gly Ser Leu Gln Val Met Asn
       50                  55                  60
   Lys Thr Arg Lys Ile Met Glu His Gly Gly Ala Thr Phe Ile Asn Ala
   65                  70                  75                  80
   Phe Val Thr Thr Pro Met Cys Cys Pro Ser Arg Ser Ser Met Leu Thr
                   85                  90                  95
   Gly Lys Tyr Val His Asn His Asn Val Tyr Thr Asn Asn Glu Asn Cys
               100                 105                 110
   Ser Ser Pro Ser Trp Gln Ala Met His Glu Pro Arg Thr Phe Ala Val
           115                 120                 125
   Tyr Leu Asn Asn Thr Gly Tyr Arg Thr Ala Phe Phe Gly Lys Tyr Leu
       130                 135                 140
   Asn Glu Tyr Asn Gly Ser Tyr Ile Pro Pro Gly Trp Arg Glu Trp Leu
   145                 150                 155                 160
   Gly Leu Ile Lys Asn Ser Arg Phe Tyr Asn Tyr Thr Val Cys Arg Asn
                   165                 170                 175
   Gly Ile Lys Glu Lys His Gly Phe Asp Tyr Ala Lys Asp Tyr Phe Thr
               180                 185                 190
   Asp Leu Ile Thr Asn Glu Ser Ile Asn Tyr Phe Lys Met Ser Lys Arg
           195                 200                 205
   Met Tyr Pro His Arg Pro Val Met Met Val Ile Ser His Ala Ala Pro
       210                 215                 220
   His Gly Pro Glu Asp Ser Ala Pro Gln Phe Ser Lys Leu Tyr Pro Asn
   225                 230                 235                 240
   Ala Ser Gln His Ile Thr Pro Ser Tyr Asn Tyr Ala Pro Asn Met Asp
                   245                 250                 255
   Lys His Trp Ile Met Gln Tyr Thr Gly Pro Met Leu Pro Ile His Met
               260                 265                 270
   Glu Phe Thr Asn Ile Leu Gln Arg Lys Arg Leu Gln Thr Leu Met Ser
           275                 280                 285
   Val Asp Asp Ser Val Glu Arg Leu Tyr Asn Met Leu Val Glu Thr Gly
       290                 295                 300
   Glu Leu Glu Asn Thr Tyr Ile Ile Tyr Thr Ala Asp His Gly Tyr His
   305                 310                 315                 320
   Ile Gly Gln Phe Gly Leu Val Lys Gly Lys Ser Met Pro Tyr Asp Phe
                   325                 330                 335
   Asp Ile Arg Val Pro Phe Phe Ile Arg Gly Pro Ser Val Glu Pro Gly
               340                 345                 350
   Ser Ile Val Pro Gln Ile Val Leu Asn Ile Asp Leu Ala Pro Thr Ile
           355                 360                 365
   Leu Asp Ile Ala Gly Leu Asp Thr Pro Pro Asp Val Asp Gly Lys Ser
       370                 375                 380
   Val Leu Lys Leu Leu Asp Pro Glu Lys Pro Gly Asn Arg Phe Arg Thr
   385                 390                 395                 400
   Asn Lys Lys Ala Lys Ile Trp Arg Asp Thr Phe Leu Val Glu Arg Gly
                   405                 410                 415
   Lys Phe Leu Arg Lys Lys Glu Glu Ser Ser Lys Asn Ile Gln Gln Ser
               420                 425                 430
   Asn His Leu Pro Lys Tyr Glu Arg Val Lys Glu Leu Cys Gln Gln Ala
           435                 440                 445
   Arg Tyr Gln Thr Ala Cys Glu Gln Pro Gly Gln Lys Trp Gln Cys Ile
       450                 455                 460
   Glu Asp Thr Ser Gly Lys Leu Arg Ile His Lys Cys Lys Gly Pro Ser
   465                 470                 475                 480
   Asp Leu Leu Thr Val Arg Gln Ser Thr Arg Asn Leu Tyr Ala Arg Gly
                   485                 490                 495
   Phe His Asp Lys Asp Lys Glu Cys Ser Cys Arg Glu Ser Gly Tyr Arg
               500                 505                 510
   Ala Ser Arg Ser Gln Arg Lys Ser Gln Arg Gln Phe Leu Arg Asn Gln
           515                 520                 525
   Gly Thr Pro Lys Tyr Lys Pro Arg Phe Val His Thr Arg Gln Thr Arg
       530                 535                 540
   Ser Leu Ser Val Glu Phe Glu Gly Glu Ile Tyr Asp Ile Asn Leu Glu
   545                 550                 555                 560
   Glu Glu Glu Glu Leu Gln Val Leu Gln Pro Arg Asn Ile Ala Lys Arg
                   565                 570                 575
   His Asp Glu Gly His Lys Gly Pro Arg Asp Leu Gln Ala Ser Ser Gly
               580                 585                 590
   Gly Asn Arg Gly Arg Met Leu Ala Asp Ser Ser Asn Ala Val Gly Pro
           595                 600                 605
   Pro Thr Thr Val Arg Val Thr His Lys Cys Phe Ile Leu Pro Asn Asp
       610                 615                 620
   Ser Ile His Cys Glu Arg Glu Leu Tyr Gln Ser Ala Arg Ala Trp Lys
   625                 630                 635                 640
   Asp His Lys Ala Tyr Ile Asp Lys Glu Ile Glu Ala Leu Gln Asp Lys
                   645                 650                 655
   Ile Lys Asn Leu Arg Glu Val Arg Gly His Leu Lys Arg Arg Lys Pro
               660                 665                 670
   Glu Glu Cys Ser Cys Ser Lys Gln Ser Tyr Tyr Asn Lys Glu Lys Gly
           675                 680                 685
   Val Lys Lys Gln Glu Lys Leu Lys Ser His Leu His Pro Phe Lys Glu
       690                 695                 700
   Ala Ala Gln Glu Val Asp Ser Lys Leu Gln Leu Phe Lys Glu Asn Asn
   705                 710                 715                 720
   Arg Arg Arg Lys Lys Glu Arg Lys Glu Lys Arg Arg Gln Arg Lys Gly
                   725                 730                 735
   Glu Glu Cys Ser Leu Pro Gly Leu Thr Cys Phe Thr His Asp Asn Asn
               740                 745                 750
   His Trp Gln Thr Ala Pro Phe Trp Asn Leu Gly Ser Phe Cys Ala Cys
           755                 760                 765
   Thr Ser Ser Asn Asn Asn Thr Tyr Trp Cys Leu Arg Thr Val Asn Glu
       770                 775                 780
   Thr His Asn Phe Leu Phe Cys Glu Phe Ala Thr Gly Phe Leu Glu Tyr
   785                 790                 795                 800
   Phe Asp Met Asn Thr Asp Pro Tyr Gln Leu Thr Asn Thr Val His Thr
                   805                 810                 815
   Val Glu Arg Gly Ile Leu Asn Gln Leu His Val Gln Leu Met Glu Leu
               820                 825                 830
   Arg Ser Cys Gln Gly Tyr Lys Gln Cys Asn Pro Arg Pro Lys Asn Leu
           835                 840                 845
   Asp Val Gly Asn Lys Asp Gly Gly Ser Tyr Asp Arg Thr Val Met Gly
       850                 855                 860
   Trp Met Gly Arg Leu Ile Ser Pro Val Ser Leu Gln Thr Ser Thr Gly
   865                 870                 875                 880
   Lys Ala
<210> 11
   <211> 1141
   <212> PRT
   <213> Homo sapiens
<400> 11
   Met Asp Asp Ser Gly Glu Leu Gly Gly Leu Glu Thr Met Glu Thr Leu
   1               5                   10                  15
   Thr Glu Leu Gly Asp Glu Leu Thr Leu Gly Asp Ile Asp Glu Met Leu
               20                  25                  30
   Gln Phe Val Ser Asn Gln Val Gly Glu Phe Pro Asp Leu Phe Ser Glu
           35                  40                  45
   Gln Leu Cys Ser Ser Phe Pro Gly Ser Gly Gly Ser Gly Ser Ser Ser
       50                  55                  60
   Gly Ser Ser Gly Ser Ser Ser Ser Ser Ser Asn Gly Arg Gly Ser Ser
   65                  70                  75                  80
   Ser Gly Ala Val Asp Pro Ser Val Gln Arg Ser Phe Thr Gln Val Thr
                   85                  90                  95
   Leu Pro Ser Phe Ser Pro Ser Ala Ala Ser Pro Gln Ala Pro Thr Leu
               100                 105                 110
   Gln Val Lys Val Ser Pro Thr Ser Val Pro Thr Thr Pro Arg Ala Thr
           115                 120                 125
   Pro Ile Leu Gln Pro Arg Pro Gln Pro Gln Pro Gln Pro Gln Thr Gln
       130                 135                 140
   Leu Gln Gln Gln Thr Val Met Ile Thr Pro Thr Phe Ser Thr Thr Pro
   145                 150                 155                 160
   Gln Thr Arg Ile Ile Gln Gln Pro Leu Ile Tyr Gln Asn Ala Ala Thr
                   165                 170                 175
   Ser Phe Gln Val Leu Gln Pro Gln Val Gln Ser Leu Val Thr Ser Ser
               180                 185                 190
   Gln Val Gln Pro Val Thr Ile Gln Gln Gln Val Gln Thr Val Gln Ala
           195                 200                 205
   Gln Arg Val Leu Thr Gln Thr Ala Asn Gly Thr Leu Gln Thr Leu Ala
       210                 215                 220
   Pro Ala Thr Val Gln Thr Val Ala Ala Pro Gln Val Gln Gln Val Pro
   225                 230                 235                 240
   Val Leu Val Gln Pro Gln Ile Ile Lys Thr Asp Ser Leu Val Leu Thr
                   245                 250                 255
   Thr Leu Lys Thr Asp Gly Ser Pro Val Met Ala Ala Val Gln Asn Pro
               260                 265                 270
   Ala Leu Thr Ala Leu Thr Thr Pro Ile Gln Thr Ala Ala Leu Gln Val
           275                 280                 285
   Pro Thr Leu Val Gly Ser Ser Gly Thr Ile Leu Thr Thr Met Pro Val
       290                 295                 300
   Met Met Gly Gln Glu Lys Val Pro Ile Lys Gln Val Pro Gly Gly Val
   305                 310                 315                 320
   Lys Gln Leu Glu Pro Pro Lys Glu Gly Glu Arg Arg Thr Thr His Asn
                   325                 330                 335
   Ile Ile Glu Lys Arg Tyr Arg Ser Ser Ile Asn Asp Lys Ile Ile Glu
               340                 345                 350
   Leu Lys Asp Leu Val Met Gly Thr Asp Ala Lys Met His Lys Ser Gly
           355                 360                 365
   Val Leu Arg Lys Ala Ile Asp Tyr Ile Lys Tyr Leu Gln Gln Val Asn
       370                 375                 380
   His Lys Leu Arg Gln Glu Asn Met Val Leu Lys Leu Ala Asn Gln Lys
   385                 390                 395                 400
   Asn Lys Leu Leu Lys Gly Ile Asp Leu Gly Ser Leu Val Asp Asn Glu
                   405                 410                 415
   Val Asp Leu Lys Ile Glu Asp Phe Asn Gln Asn Val Leu Leu Met Ser
               420                 425                 430
   Pro Pro Ala Ser Asp Ser Gly Ser Gln Ala Gly Phe Ser Pro Tyr Ser
           435                 440                 445
   Ile Asp Ser Glu Pro Gly Ser Pro Leu Leu Asp Asp Ala Lys Val Lys
       450                 455                 460
   Asp Glu Pro Asp Ser Pro Pro Val Ala Leu Gly Met Val Asp Arg Ser
   465                 470                 475                 480
   Arg Ile Leu Leu Cys Val Leu Thr Phe Leu Cys Leu Ser Phe Asn Pro
                   485                 490                 495
   Leu Thr Ser Leu Leu Gln Trp Gly Gly Ala His Asp Ser Asp Gln His
               500                 505                 510
   Pro His Ser Gly Ser Gly Arg Ser Val Leu Ser Phe Glu Ser Gly Ser
           515                 520                 525
   Gly Gly Trp Phe Asp Trp Met Met Pro Thr Leu Leu Leu Trp Leu Val
       530                 535                 540
   Asn Gly Val Ile Val Leu Ser Val Phe Val Lys Leu Leu Val His Gly
   545                 550                 555                 560
   Glu Pro Val Ile Arg Pro His Ser Arg Ser Ser Val Thr Phe Trp Arg
                   565                 570                 575
   His Arg Lys Gln Ala Asp Leu Asp Leu Ala Arg Gly Asp Phe Ala Ala
   580 585 590
   Ala Ala Gly Asn Leu Gln Thr Cys Leu Ala Val Leu Gly Arg Ala Leu
   595 600 605
   Pro Thr Ser Arg Leu Asp Leu Ala Cys Ser Leu Ser Trp Asn Val Ile
   610 615 620
   Arg Tyr Ser Leu Gln Lys Leu Arg Leu Val Arg Trp Leu Leu Lys Lys
   625 630 635 640
   Val Phe Gln Cys Arg Arg Ala Thr Pro Ala Thr Glu Ala Gly Phe Glu
   645 650 655
   Asp Glu Ala Lys Thr Ser Ala Arg Asp Ala Ala Leu Ala Tyr His Arg
   660 665 670
   Leu His Gln Leu His Ile Thr Gly Lys Leu Pro Ala Gly Ser Ala Cys
   675 680 685
   Ser Asp Val His Met Ala Leu Cys Ala Val Asn Leu Ala Glu Cys Ala
   690 695 700
   Glu Glu Lys Ile Pro Pro Ser Thr Leu Val Glu Ile His Leu Thr Ala
   705 710 715 720
   Ala Met Gly Leu Lys Thr Arg Cys Gly Gly Lys Leu Gly Phe Leu Ala
   725 730 735
   Ser Tyr Phe Leu Ser Arg Ala Gln Ser Leu Cys Gly Pro Glu His Ser
   740 745 750
   Ala Val Pro Asp Ser Leu Arg Trp Leu Cys His Pro Leu Gly Gln Lys
   755 760 765
   Phe Phe Met Glu Arg Ser Trp Ser Val Lys Ser Ala Ala Lys Glu Ser
   770 775 780
   Leu Tyr Cys Ala Gln Arg Asn Pro Ala Asp Pro Ile Ala Gln Val His
   785 790 795 800
   Gln Ala Phe Cys Lys Asn Leu Leu Glu Arg Ala Ile Glu Ser Leu Val
   805 810 815
   Lys Pro Gln Ala Lys Lys Lys Ala Gly Asp Gln Glu Glu Glu Ser Cys
   820 825 830
   Glu Phe Ser Ser Ala Leu Glu Tyr Leu Lys Leu Leu His Ser Phe Val
   835 840 845
   Asp Ser Val Gly Val Met Ser Pro Pro Leu Ser Arg Ser Ser Val Leu
   850 855 860
   Lys Ser Ala Leu Gly Pro Asp Ile Ile Cys Arg Trp Trp Thr Ser Ala
   865 870 875 880
   Ile Thr Val Ala Ile Ser Trp Leu Gln Gly Asp Asp Ala Ala Val Arg
   885 890 895
   Ser His Phe Thr Lys Val Glu Arg Ile Pro Lys Ala Leu Glu Val Thr
   900 905 910
   Glu Ser Pro Leu Val Lys Ala Ile Phe His Ala Cys Arg Ala Met His
   915 920 925
   Ala Ser Leu Pro Gly Lys Ala Asp Gly Gln Gln Ser Ser Phe Cys His
   930 935 940
   Cys Glu Arg Ala Ser Gly His Leu Trp Ser Ser Leu Asn Val Ser Gly
   945 950 955 960
   Ala Thr Ser Asp Pro Ala Leu Asn His Val Val Gln Leu Leu Thr Cys
   965 970 975
   Asp Leu Leu Leu Ser Leu Arg Thr Ala Leu Trp Gln Lys Gln Ala Ser
   980 985 990
   Ala Ser Gln Ala Val Gly Glu Thr Tyr His Ala Ser Gly Ala Glu Leu
   995 1000 1005
   Ala Gly Phe Gln Arg Asp Leu Gly Ser Leu Arg Arg Leu Ala His
   1010 1015 1020
   Ser Phe Arg Pro Ala Tyr Arg Lys Val Phe Leu His Glu Ala Thr
   1025 1030 1035
   Val Arg Leu Met Ala Gly Ala Ser Pro Thr Arg Thr His Gln Leu
   1040 1045 1050
   Leu Glu His Ser Leu Arg Arg Arg Thr Thr Gln Ser Thr Lys His
   1055 1060 1065
   Gly Glu Val Asp Ala Trp Pro Gly Gln Arg Glu Arg Ala Thr Ala
   1070 1075 1080
   Ile Leu Leu Ala Cys Arg His Leu Pro Leu Ser Phe Leu Ser Ser
   1085 1090 1095
   Pro Gly Gln Arg Ala Val Leu Leu Ala Glu Ala Ala Arg Thr Leu
   1100 1105 1110
   Glu Lys Val Gly Asp Arg Arg Ser Cys Asn Asp Cys Gln Gln Met
   1115 1120 1125
   Ile Val Lys Leu Gly Gly Gly Thr Ala Ile Ala Ala Ser
   1130 1135 1140
<210> 12
   <211> 976
   <212> PRT
   <213> Homo sapiens
<400> 12
   Met Ala Gln Val Ala Met Ser Thr Leu Pro Val Glu Asp Glu Glu Ser
   1 5 10 15
   Ser Glu Ser Arg Met Val Val Thr Phe Leu Met Ser Ala Leu Glu Ser
   20 25 30
   Met Cys Lys Glu Leu Ala Lys Ser Lys Ala Glu Val Ala Cys Ile Ala
   35 40 45
   Val Tyr Glu Thr Asp Val Phe Val Val Gly Thr Glu Arg Gly Arg Ala
   50 55 60
   Phe Val Asn Thr Arg Lys Asp Phe Gln Lys Asp Phe Val Lys Tyr Cys
   65 70 75 80
   Val Glu Glu Glu Glu Lys Ala Ala Glu Met His Lys Met Lys Ser Thr
   85 90 95
   Thr Gln Ala Asn Arg Met Ser Val Asp Ala Val Glu Ile Glu Thr Leu
   100 105 110
   Arg Lys Thr Val Glu Asp Tyr Phe Cys Phe Cys Tyr Gly Lys Ala Leu
   115 120 125
   Gly Lys Ser Thr Val Val Pro Val Pro Tyr Glu Lys Met Leu Arg Asp
   130 135 140
   Gln Ser Ala Val Val Val Gln Gly Leu Pro Glu Gly Val Ala Phe Lys
   145 150 155 160
   His Pro Glu Asn Tyr Asp Leu Ala Thr Leu Lys Trp Ile Leu Glu Asn
   165 170 175
   Lys Ala Gly Ile Ser Phe Ile Ile Lys Arg Pro Phe Leu Glu Pro Lys
   180 185 190
   Lys His Val Gly Gly Arg Val Met Val Thr Asp Ala Asp Arg Ser Ile
   195 200 205
   Leu Ser Pro Gly Gly Ser Cys Gly Pro Ile Lys Val Lys Thr Glu Pro
   210 215 220
   Thr Glu Asp Ser Gly Ile Ser Leu Glu Met Ala Ala Val Thr Val Lys
   225 230 235 240
   Glu Glu Ser Glu Asp Pro Asp Tyr Tyr Gln Tyr Asn Ile Gln Gly Pro
   245 250 255
   Ser Glu Thr Asp Asp Val Asp Glu Lys Gln Pro Leu Ser Lys Pro Leu
   260 265 270
   Gln Gly Ser His His Ser Ser Glu Gly Asn Glu Gly Thr Glu Met Glu
   275 280 285
   Val Pro Ala Glu Asp Asp Asp Tyr Ser Pro Pro Ser Lys Arg Pro Lys
   290 295 300
   Ala Asn Glu Leu Pro Gln Pro Pro Val Pro Glu Pro Ala Asn Ala Gly
   305 310 315 320
   Lys Arg Lys Val Arg Glu Phe Asn Phe Glu Lys Trp Asn Ala Arg Ile
   325 330 335
   Thr Asp Leu Arg Lys Gln Val Glu Glu Leu Phe Glu Arg Lys Tyr Ala
   340 345 350
   Gln Ala Ile Lys Ala Lys Gly Pro Val Thr Ile Pro Tyr Pro Leu Phe
   355 360 365
   Gln Ser His Val Glu Asp Leu Tyr Val Glu Gly Leu Pro Glu Gly Ile
   370 375 380
   Pro Phe Arg Arg Pro Ser Thr Tyr Gly Ile Pro Arg Leu Glu Arg Ile
   385 390 395 400
   Leu Leu Ala Lys Glu Arg Ile Arg Phe Val Ile Lys Lys His Glu Leu
   405 410 415
   Leu Asn Ser Thr Arg Glu Asp Leu Gln Leu Asp Lys Pro Ala Ser Gly
   420 425 430
   Val Lys Glu Glu Trp Tyr Ala Arg Ile Thr Lys Leu Arg Lys Met Val
   435 440 445
   Asp Gln Leu Phe Cys Lys Lys Phe Ala Glu Ala Leu Gly Ser Thr Glu
   450 455 460
   Ala Lys Ala Val Pro Tyr Gln Lys Phe Glu Ala His Pro Asn Asp Leu
   465 470 475 480
   Tyr Val Glu Gly Leu Pro Glu Asn Ile Pro Phe Arg Ser Pro Ser Trp
   485 490 495
   Tyr Gly Ile Pro Arg Leu Glu Lys Ile Ile Gln Val Gly Asn Arg Ile
   500 505 510
   Lys Phe Val Ile Lys Arg Pro Glu Leu Leu Thr His Ser Thr Thr Glu
   515 520 525
   Val Thr Gln Pro Arg Thr Asn Thr Pro Val Lys Glu Asp Trp Asn Val
   530 535 540
   Arg Ile Thr Lys Leu Arg Lys Gln Val Glu Glu Ile Phe Asn Leu Lys
   545 550 555 560
   Phe Ala Gln Ala Leu Gly Leu Thr Glu Ala Val Lys Val Pro Tyr Pro
   565 570 575
   Val Phe Glu Ser Asn Pro Glu Phe Leu Tyr Val Glu Gly Leu Pro Glu
   580 585 590
   Gly Ile Pro Phe Arg Ser Pro Thr Trp Phe Gly Ile Pro Arg Leu Glu
   595 600 605
   Arg Ile Val Arg Gly Ser Asn Lys Ile Lys Phe Val Val Lys Lys Pro
   610 615 620
   Glu Leu Val Ile Ser Tyr Leu Pro Pro Gly Met Ala Ser Lys Ile Asn
   625 630 635 640
   Thr Lys Ala Leu Gln Ser Pro Lys Arg Pro Arg Ser Pro Gly Ser Asn
   645 650 655
   Ser Lys Val Pro Glu Ile Glu Val Thr Val Glu Gly Pro Asn Asn Asn
   660 665 670
   Asn Pro Gln Thr Ser Ala Val Arg Thr Pro Thr Gln Thr Asn Gly Ser
   675 680 685
   Asn Val Pro Phe Lys Pro Arg Gly Arg Glu Phe Ser Phe Glu Ala Trp
   690 695 700
   Asn Ala Lys Ile Thr Asp Leu Lys Gln Lys Val Glu Asn Leu Phe Asn
   705                 710                 715                 720
   Glu Lys Cys Gly Glu Ala Leu Gly Leu Lys Gln Ala Val Lys Val Pro
                   725                 730                 735
   Phe Ala Leu Phe Glu Ser Phe Pro Glu Asp Phe Tyr Val Glu Gly Leu
               740                 745                 750
   Pro Glu Gly Val Pro Phe Arg Arg Pro Ser Thr Phe Gly Ile Pro Arg
           755                 760                 765
   Leu Glu Lys Ile Leu Arg Asn Lys Ala Lys Ile Lys Phe Ile Ile Lys
       770                 775                 780
   Lys Pro Glu Met Phe Glu Thr Ala Ile Lys Glu Ser Thr Ser Ser Lys
   785                 790                 795                 800
   Ser Pro Pro Arg Lys Ile Asn Ser Ser Pro Asn Val Asn Thr Thr Ala
                   805                 810                 815
   Ser Gly Val Glu Asp Leu Asn Ile Ile Gln Val Thr Ile Pro Asp Asp
               820                 825                 830
   Asp Asn Glu Arg Leu Ser Lys Val Glu Lys Ala Arg Gln Leu Arg Glu
           835                 840                 845
   Gln Val Asn Asp Leu Phe Ser Arg Lys Phe Gly Glu Ala Ile Gly Met
       850                 855                 860
   Gly Phe Pro Val Lys Val Pro Tyr Arg Lys Ile Thr Ile Asn Pro Gly
   865                 870                 875                 880
   Cys Val Val Val Asp Gly Met Pro Pro Gly Val Ser Phe Lys Ala Pro
                   885                 890                 895
   Ser Tyr Leu Glu Ile Ser Ser Met Arg Arg Ile Leu Asp Ser Ala Glu
               900                 905                 910
   Phe Ile Lys Phe Thr Val Ile Arg Pro Phe Pro Gly Leu Val Ile Asn
           915                 920                 925
   Asn Gln Leu Val Asp Gln Ser Glu Ser Glu Gly Pro Val Ile Gln Glu
       930                 935                 940
   Ser Ala Glu Pro Ser Gln Leu Glu Val Pro Ala Thr Glu Glu Ile Lys
   945                 950                 955                 960
   Glu Thr Asp Gly Ser Ser Gln Ile Lys Gln Glu Pro Asp Pro Thr Trp
                   965                 970                 975
<210> 13
   <211> 21
   <212> DNA
   <213> Artificial
<220>
   <223> Cebador directo T7_up2
<400> 13
   tgctaaggac aacgttatcg g 21
<210> 14
   <211> 18
   <212> DNA
   <213> Artificial
<220>
   <223> Cebador inverso T7_down2
<400> 14
   ttgataccgg acgttcac 18

## Description

### Field of the Invention

The present invention is comprised within the field of biomedicine. It specifically relates to a method for detecting an autoantibody in a sample and to a kit for carrying out said method.

### Background of the Invention

Colorectal cancer (CRC) is the second most prevalent cancer in the Western world. The disease develops over decades and involves multiple genetic events. Despite the fact that CRC is one of the best characterized solid tumors from a genetic viewpoint, it continues to be one of the main causes of death in developed countries because of the late diagnosis of patients due to the waiting time that passes to perform certain diagnostic tests, such as colonoscopy.

Today there are few proteins that have been described as effective biomarkers of CRC (carcinoembryonic antigen (CEA), CA19.9 and CA125) (Crawford et al. 2003. Journal of surgical oncology 84 (4), 239-248; Duffy et al. 2007 Eur J Cancer 43 (9), 1348-1360) and they are not specific enough to perform clinical screenings with a view to detect CRC (Locker et al. 25 2006. J Clin Oncol 24 (33), 5313-5327).

Proteomic analyses are being actively used for identifying new biomarkers. In different earlier proteomic studies, differentially expressed proteins in CRC tissue have been identified by means of using antibody microarrays and 2D-DIGE, including isoforms and post-transductional modifications responsible for modifications in signaling pathways (Alfonso et al. 2005. Proteomics 5(10), 2602-2611; Kopf et al. 2005. Proteomics 5(9), 2412-2416; Madoz-Gurpide et al. 2007. Mol Cell Proteomics 6 (12), 2150-2164; Alfonso et al. 2008. Journal of Proteome Research 7 (10), 4247-4255). These two approaches have allowed identifying a broad collection of potential tumor markers of CRC tissue which are currently under research.

However, the implementation of non-invasive and simpler diagnostic methods which allow early detection of CRC must be based on identifying proteins or antibodies detectable in serum or plasma (Hanash et al. 2008. Nature 452 (7187), 571-579; Hudson et al. 2007. Proceedings of the National Academy of Sciences of the United States of America 104 (44), 17494-17499). The existence of an immune response to cancer in humans has been shown by the presence of autoantibodies in serum from cancer patients. Different human proteins (autoantigens) can thereby be affected before or during the formation of the tumor, being able to produce an immune response once released (Hudson et al. 2007. Proceedings of the National Academy of Sciences of the United States of America 104 (44), 17494-17499; Wang et al. 2005. The New England Journal of Medicine 353 (12), 1224-1235; Sreekumar et al. 2004. J Natl Cancer Inst 96 (11), 834-843). Said autoantibodies can be detected in early stages of the disease and even before the cancer can be detected by means of other techniques, indicating their enormous potential as biomarkers of the disease. These tumor proteins can be affected by point mutations, have anomalous folding, be overexpressed, aberrantly glycosylate, be truncated or undergo aberrant degradation as is the case of p53, HER2, NY-ESO1 or MUC1, respectively (Chen et al. 1997. Proceedings of the National Academy of Sciences of the United States of America 94 (5), 1914-1918; Schubert et al. 2000. Nature 404 (6779), 770-774; Ulanet et al. 2003. Proceedings of the National Academy of Sciences of the United States of America 100 (21), 12361-12366). In fact, tumor-associated autoantigens (TAAs) have previously been characterized in CRC using different approaches (Scanlan et al. 1998. International Journal of Cancer 76 (5), 652-658). Several authors have described some TAA panels as biomarkers of CRC, among which STK4/MST1 protein is found (Tan et al. 2009. Journal 276: 6880-6904; Babel et al. 2009. Molecular and Cellular Proteomics 8: 2382-2395; WO 2010/136629). WO2009/008496 discloses that hSULF1 is highly expressed in colon cancer and an antigen that can be used for the production of an anti-hSULFl antibody which inhibits cell proliferation of a cancer cell. Nevertheless, the diagnostic validity of the autoantibodies associated with CRC identified until now still requires an independent validation for their generalized use in the diagnosis/prognosis of CRC.

Therefore, there is a need for biomarkers which allow the diagnosis of CRC, its classification in the different stages of tumor progression, the prognosis of disease progression, the evaluation of its response to a specific treatment and the detection of the recurrence or the spread of CRC, by means of a simple, effective and non-invasive method.

### Brief Description of the Invention

The present disclosure relates to a method for obtaining useful data for the diagnosis, prognosis or monitoring of colorectal cancer (CRC) progression, to a method for the diagnosis of CRC, to a method for the prognosis of CRC and to a kit for carrying out said methods.

The present disclosure therefore provides a response to the need for biomarkers which allow the diagnosis of CRC, its classification in the different stages of tumor progression, the prognosis of disease progression, the evaluation of its response to a specific treatment and the detection of the recurrence or the spread of CRC, by means of a simple, effective and non-invasive method.

Blood is usually the optimal biological fluid based on non-invasive methods for massive screening of large populations of patients for diagnostic purposes. On one hand, serum and plasma are easy to obtain, and on the other hand, blood circulation facilitates the contact of the blood with all the tissues of the human body, including contact with tumor tissue and its representative antigens in the case of cancer patients. The release of these tumor associated antigens probably occurs at a very low concentration in plasma and probably experience proteolysis in a short time period. In contrast, antibodies are very stable molecules which have been used for years in different clinical immunoassays, which facilitates standardizing assays. The use of autoantibodies is also beneficial because the immune system amplifies the response, facilitating identification and quantification.

Phage microarrays (occasionally identified in this description as "phages-peptides") have been used in the present invention to identify autoantibodies present in serum from CRC patients at different stages.

Six phages containing sequences homologous to NHSL1, GRN, MST1, SULF1, SREBF2 and GTF2i proteins were selected. The combination of MST1 and SULF1 recombinant proteins with the 4 other phage sequences allowed predicting the disease with 72% sensitivity and 87% specificity, with an Area Under the Curve (AUC) of 0.83. If the age of the patient is further taken into account, the AUC is 0.91. These markers further allow grouping the results by discriminating not only the sick individuals but also the different stages of the disease. The detection of this panel of autoantibodies in serum is therefore a simple and non-invasive method for the diagnosis/prognosis of CRC.

In a first aspect, the invention relates to a method for detecting an autoantibody in a sample, comprising:
a) contacting a sample from a subject with an antibody capturing entity (ACE), wherein said ACE is an ACE comprising the amino acid sequence shown in SEQ ID NO: 4 or a peptide or protein having at least 90% sequence identity with respect to SEQ ID NO: 4 and that is recognized by an autoantibody against SEQ ID NO: 4;
   and
b) detecting the formation of a complex between an autoantibody and said ACE,
   wherein the detection of said autoantibody-ACE complex is indicative of the presence of said autoantibody in said sample, wherein the sample is blood, plasma or serum.

In a second aspect, the invention relates to an antibody capturing entity (ACE) comprising the amino acid sequence shown in SEQ ID NO: 4, wherein said ACE is not sulfatase-1 (SULF1) protein.

In a third aspect, the invention relates to a composition comprising an antibody capturing entity (ACE) according to the invention.

In a fourth aspect, the invention relates to a kit comprising a composition according to the invention.

In a fifth aspect, the invention relates to an in vitro use of a kit according to the invention for detecting an antibody in a sample, or for detecting an autoantibody in a subject suspected of having colorectal cancer (CRC), or for diagnosing whether a subject has CRC, or for determining the risk of a subject developing CRC, or for monitoring CRC progression in a subject, or for evaluating the efficacy of a treatment against CRC, or for predicting survival of a subject who has CRC.

### Brief Description of the Drawings

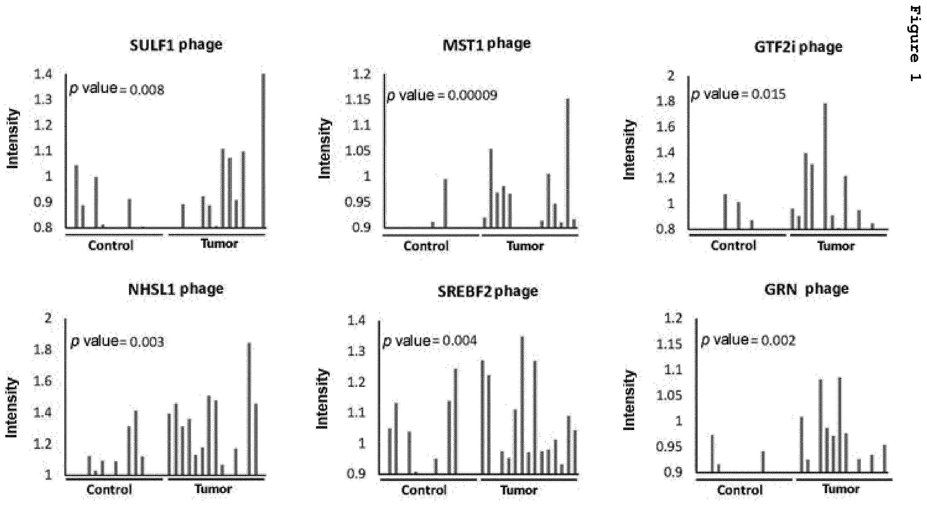

Figure 1 shows the response of the autoantibodies to the six specific phages. Intensity of the signal of each phage with the CRC sera and the control sera. The results show the data after normalization and on a scale of arbitrary units (a.u.).

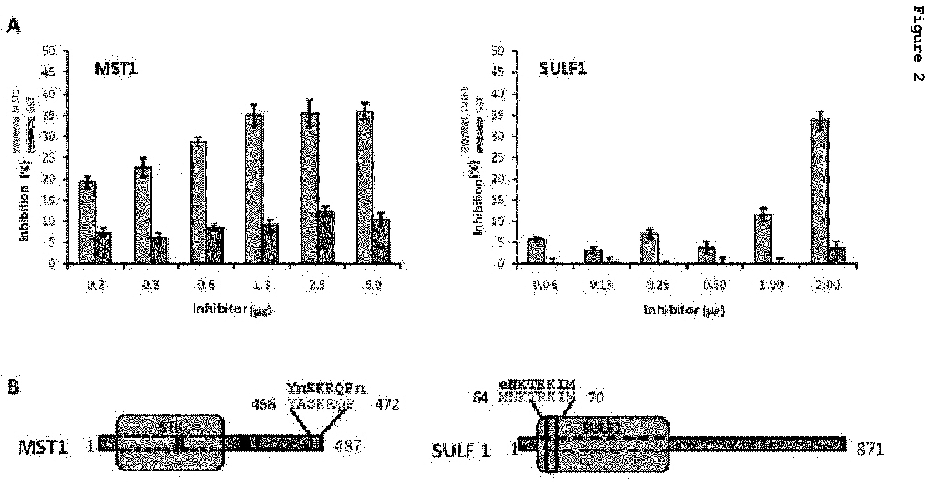

Figure 2 shows the competitive analysis between the phage peptides and their respective homologous proteins. A. An ELISA competitive assay was conducted between the phages displaying peptides with homology to SULF1 and MST1 and their respective recombinant proteins. GST was used as a negative inhibition control. Increasing amounts of the recombinant proteins were pre-incubated with the serum from patients and their respective phage binding was tested by means of ELISA (Vertical bars: light gray, recombinant protein; dark gray, GST). EBNA1 protein was used as a control to demonstrate that inhibition was specific of the protein used as inhibitor and that no error was introduced in the assay (data not shown). B. Schematic location of the peptides with homology to SULF1 and MST1 within the recombinant proteins. The position of the peptide in the protein is highlighted in the figure. The vertical lines correspond to potential phosphorylation sites. The different amino acids between the phage and protein sequence are in lower case letters.

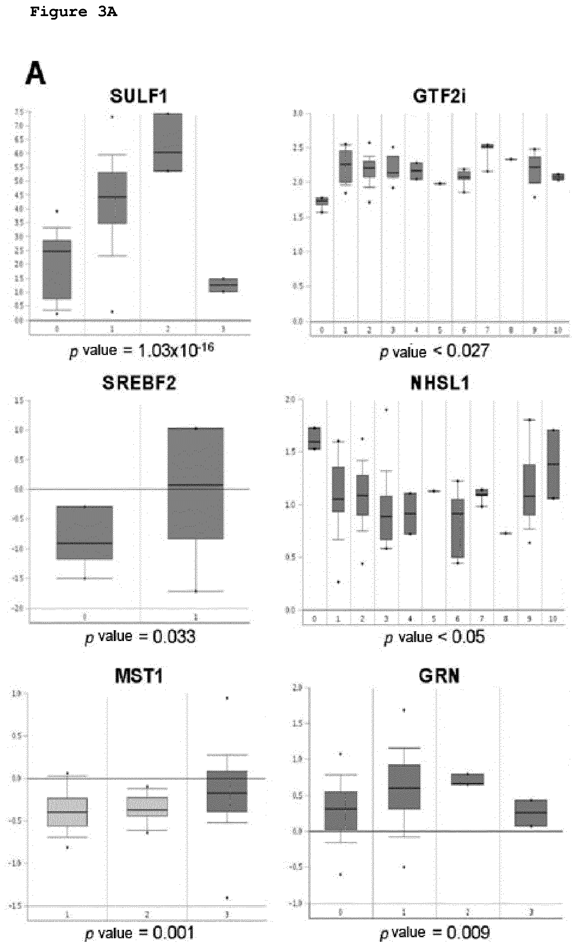

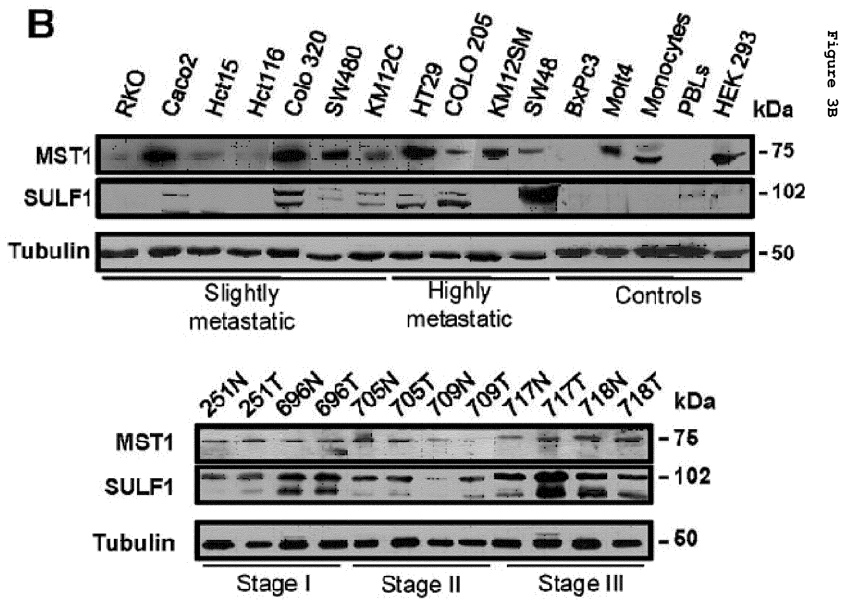

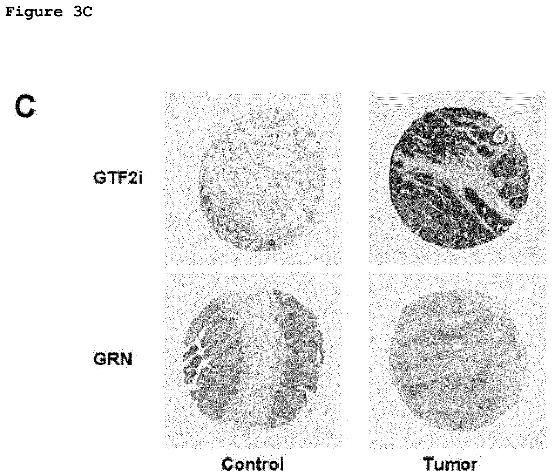

Figure 3 shows the analysis of SULF1, MST1, GTF2i, NHSL1, GRN and SREBF2 expression in cell lines and tissue from CRC patients. A. The gene expression levels of the proteins the peptide of which is displayed in T7 phages was determined by means of meta-analysis using the Oncomine database. The p values are indicated in the figure. Relative gene expression levels were found for NHSL1, SREBF2, GTF2i, SULF1, MST1 and GRN. B. Immunodetection on membrane of SULF1 and MST1 in colorectal cancer cell lines compared with control cell lines and paired tissues of CRC belonging to stages I, II and III. An anti-tubulin antibody was used as a loading control. C, Data of the tissue microarray for GTF2i and GRN obtained from the Human Protein Atlas WebPage.

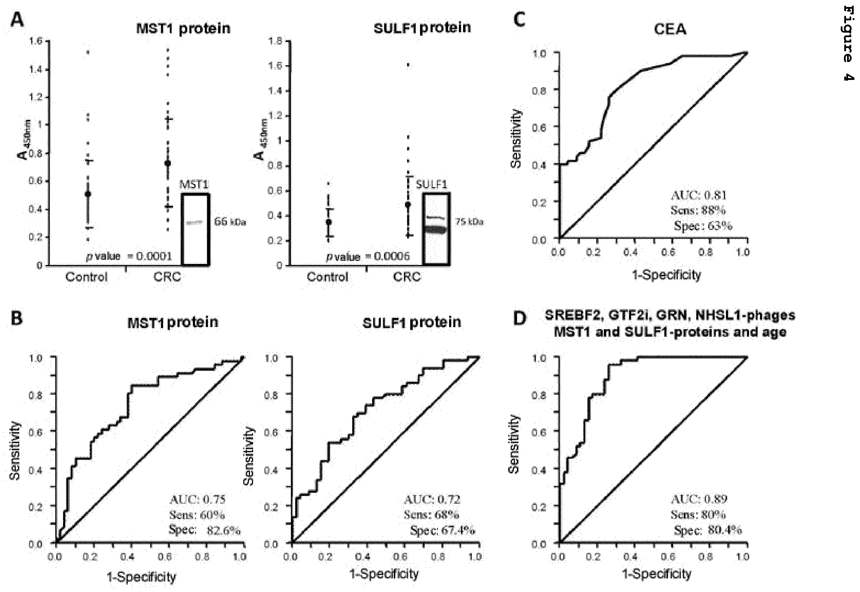

Figure 4 shows the predictive values of MST1 and SULF1 proteins. A. Mean absorbance values obtained with CRC sera and the controls by means of indirect ELISA. The dots represent the individual value for each serum. The error bars represent the standard deviation value. The images of the polyacrylamide gels correspond to the recombinant proteins used in the ELISA assays. B. Both proteins were capable of discriminating control sera from sera from CRC patients with p values < 0.0001 and 0.0006 for MST1 and SULF1, respectively. The AUC for MST1 was 0.75 (95% CI= 0.647-0.829) with sensitivity and specificity of 60.0% and 82.6%, respectively, using 0.63 as a cut-off point. The AUC was 0.72 (95% CI= 0.617-0.805) for SULF1, with sensitivity and specificity of 68% and 67.4%, respectively, using 0.36 as a cutoff point. C and D, ROC curves for CEA and the combination of 4 phages, 2 proteins and the age variable, resulting in AUC values of 0.81 and 0.89, respectively.

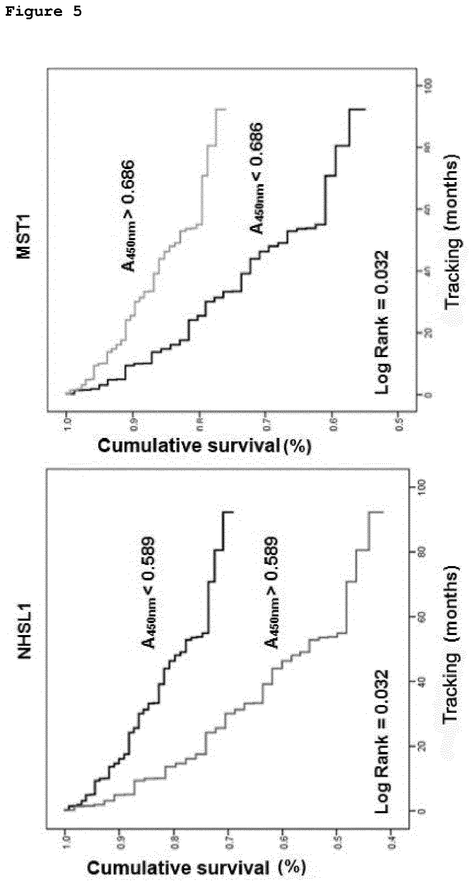

Figure 5 shows the survival analysis using autoantibodies against MST1 and NHSL1 proteins. The Kaplan-Meier survival curves were calculated using an independent set of 95 sera from CRC patients to analyze the effect of the presence of autoantibodies on absolute survival of CRC patients.

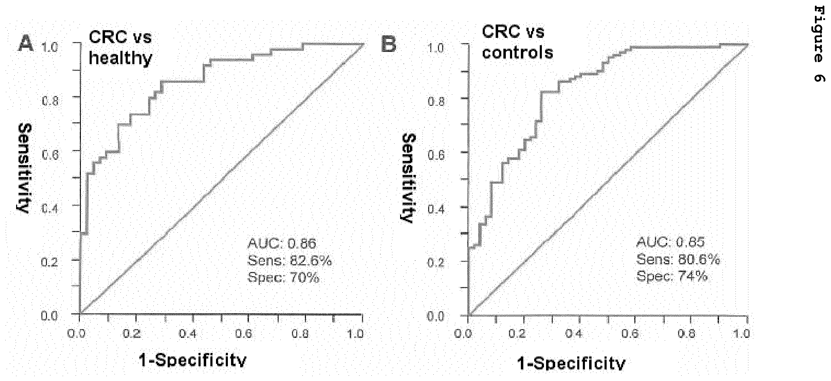

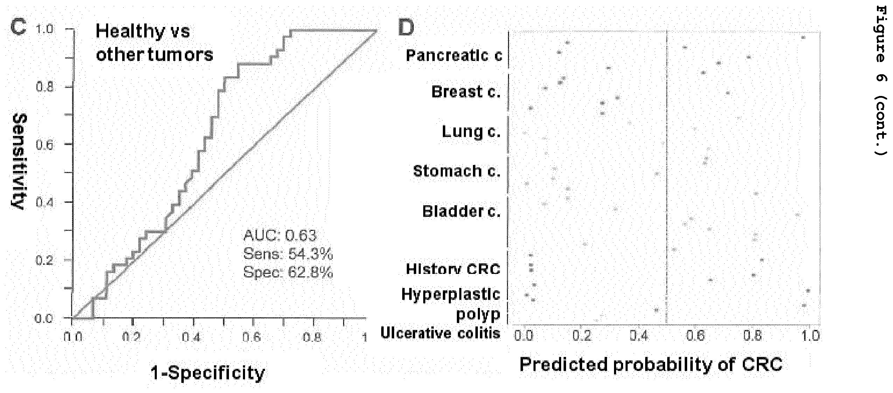

Figure 6 shows the validation of the combination of four phages with MST1 and SULF1 proteins in the diagnosis of colorectal cancer. Behavior of the combination of MST1 and NHSLlphages GTF2i, NHSL1, GRN and SREBF2 and MST1 and NHSL1 proteins in the validation test. A. Behavior of CRC samples versus healthy controls. B. Behavior of CRC samples versus reference sera. C. Behavior of healthy sera versus tumor sera. D. Dotplot showing the individual probability of being classified as a CRC patient for each of the subjects with different pathologies. Most of the samples were classified below the probability value of 0.5 (dotted vertical line).

### Detailed Description of the Invention

### Definitions

The meaning of some terms and expressions as they are used in the present description are indicated below to aid in understanding.

As used herein, the term "antibody" refers to immunoglobulin molecules and immunologically active portions of immunoglobulin molecules, i.e., molecules containing an antigen fixing site binding specifically (immunoreacting) with an antigen, such as a protein for example. There are 5 isotypes or main classes of immunoglobulins: immunoglobulin M (IgM), immunoglobulin D (IgD), immunoglobulin G (IgG), immunoglobulin A (IgA) and immunoglobulin E (IgE).

As used herein, the term "autoantibody" applies to an antibody reacting against an antigen present in the subject's organism, even if the reaction occurs only *in vitro,* and whether or not it causes pathological effects *in vivo.*

As used herein, the term "colorectal cancer" or "CRC", also called colon cancer, includes any type of neoplasias of the colon, rectum and appendix, as well as any histological subtype typically occurring in colon cancer, e.g., transitional carcinoma cells, squamous carcinoma and adenocarcinoma cells, any clinical subtype, e.g., surface, invasive muscle or metastatic disease cancer, or any TNM stage including T0-T4, N0-N2 and M0-M1 tumors. Patients can be classified in different groups with respect to the stage of the tumor. The classification of colon cancer is an estimate of the penetration of a particular cancer. It is carried out for investigational purposes, diagnostic purposes and for determining the best method of treatment. The system for the classification of colorectal cancer depends on the extent of local invasion, on the degree of lymphatic nodes involved and on if distant metastasis exists. The most common classification system is the TNM (for tumors/nodes/metastasis) system, of the American Joint Committee on Cancer (AJCC). The TNM system assigns a number based on three categories. "T" indicates the degree of invasion of the intestinal wall, "N" the degree of involvement of lymphatic nodes and "M" the degree of metastasis. The broadest stage of cancer is usually mentioned as a number I, II, III, IV derived from the TNM value grouped by the prognosis, a higher number indicates a more advanced cancer and a worse prognosis. Details of the classification are indicated in Table 1.

**Table 1**

| TNM system for the classification of CRC | | |
|---|---|---|
| **AJCC Stage** | **TNM Stage** | **Criteria of TNM stages for CRC** |
| Stage 0 | Tis N0 M0 | Tis: The tumor confined to the mucosa; cancer-*in-situ* |
| Stage I | T1 N0 M0 | T1: The tumor invades the mucosa |
| Stage I | T2 N0 M0 | T2: The tumor invades the actual muscles |
| Stage II-A | T3 N0 M0 | T3: The tumor invades the subserosal layer or beyond (other organs not involved) |
| Stage II-B | T4 N0 M0 | T4: The tumor invades adjacent organs or perforates the visceral peritoneum |
| Stage III-A | T1-2 N1 M0 | N1: Metastasis of 1 to 3 regional lymphatic nodes. T1 or T2. |
| Stage III-B | T3-4 N1 M0 | N1: Metastasis of 1 to 3 regional lymphatic nodes. T3 or T4. |
| Stage III-C | any T, N2 M0 | N2: Metastasis of 4 or more regional lymphatic nodes. Any T. |
| Stage IV | any T, any N, M1 | M1: Presence of distant metastasis. Any T, any N. |

As used herein, the term "antibody capturing entity" (ACE) refers to a macromolecular entity binding specifically to an antibody (or autoantibody). In a particular disclosure, said ACE comprises a peptide or a protein binding specifically to an antibody (or autoantibody). Said peptide can either be immobilized on a support or exposed on the phage surface. In a preferred particular disclosure, said ACE is a peptide, a protein or a phage on the surface of which said peptide or said protein is exposed. If desired, said ACE can be immobilized on a solid support.

As used herein, the term "sample" refers but is not limited to tissues and/or biological fluids from a subject, obtained by means of any method known by a person skilled in the art which serves for carrying out any of the methods provided by the present disclosure; i.e., said biological sample must be a sample susceptible of containing antibodies, e.g., autoantibodies against SULF1, MST1 proteins, etc., or against ACEs comprising the amino acid sequences shown in SEQ ID NO: 1-6, or variants thereof containing epitopes recognizable by autoantibodies, etc. By way of non-limiting illustration, said biological sample can be a blood, urine, saliva, serum, or plasma sample, a buccal or buccal-pharyngeal swab, a surgical specimen, a specimen obtained from a biopsy or autopsy, etc. In a particular disclosure, said sample is a biological fluid. In a preferred disclosure for the detection of autoantibodies, the sample from the subject is blood, plasma or blood serum. In another particular disclosure, said sample is a tissue sample. In a preferred disclosure for the quantification of SULF1 protein level, said sample is preferably a colorectal tissue sample or tumor tissue sample, etc., obtained by conventional methods, for example, by means of a biopsy, resection, etc.

As used herein, the term "SULF1 protein" includes SULF1 protein and variants thereof; in a particular disclosure, said protein is the protein with NCBI database accession number (May 1, 2011 version) EAW86954.1 and its amino acid sequence is SEQ ID NO: 10.

As used herein, the term "NHSL1 protein" includes NHSL1 protein and variants thereof; in a particular disclosure, said protein is the protein with NCBI database accession number (May 1, 2011 version) NP_001137532.1 and its amino acid sequence is SEQ ID NO: 7.

As used herein, the term "GRN protein" includes GRN protein and variants thereof; in a particular disclosure, said protein is the protein with NCBI database accession number (May 1, 2011 version) 2JYT and its amino acid sequence is SEQ ID NO: 8.

As used herein, the term "MST1 protein" includes MST1 protein and variants thereof; in a particular disclosure, said protein is the protein with NCBI database accession number (May 1, 2011 version) AAA83254.1 and its amino acid sequence is SEQ ID NO: 9.

As used herein, the term "SREBF2 protein" includes SREBF2 protein and variants thereof; in a particular embdisclosureodiment, said protein is the protein with NCBI database accession number (May 1, 2011 version) NP_004590.2 and its amino acid sequence is SEQ ID NO: 11.

As used herein, the term "GTF2i protein" includes GTF2i protein and variants thereof; in a particular disclosure, said protein is the protein with NCBI database accession number (May 1, 2011 version) NP_001157108.1 and its amino acid sequence is SEQ ID NO: 12.

As used herein, the term "variant" refers to a protein or peptide substantially homologous to another protein or peptide, for example, to the peptides the amino acid sequences of which are shown in SEQ ID NO:1 to 6, to SULF1, MST1, NHSL1, GRN, SREBF2 or GTF2i proteins, etc. A variant generally includes additions, deletions or substitutions of one or more amino acids. The person skilled in the art will understand that the amino acid sequences referred to in this description can be chemically modified, for example, by means of physiologically relevant chemical modifications, such as phosphorylations, acetylations, glycosylations or methylations. According to the present disclosure, said variants are recognized by autoantibodies against the protein or peptide in question. Variants of said peptides or proteins include peptides or proteins showing at least 25%, at least 40%, at least 60%, at least 70%, at least 80%, at least 90%, at least 95%, at least 96%, at least 97%, at least 98% or at least 99% sequence identity with respect to certain amino acid sequences of peptides or proteins. The degree of identity between two amino acid sequences can be determined by conventional methods, for example, by means of standard sequence alignment algorithms known in the state of the art, such as BLAST for example (Altschul S.F. et al. Basic local alignment search tool. J Mol Biol. 1990; 215(3):403-10).

### Method for detecting autoantibodies in subjects with CRC

It is disclosed a method for detecting an autoantibody in a subject suspected of having colorectal cancer (CRC), hereinafter first method of the disclosure, comprising:
a) contacting a sample from said subject with an antibody capturing entity (ACE), wherein said ACE is selected from the group consisting of:
   (i) an ACE comprising the amino acid sequence shown in SEQ ID NO: 4 or a variant thereof containing an epitope recognizable by an autoantibody;
   (ii) an ACE comprising the amino acid sequence shown in SEQ ID NO: 2 or a variant thereof containing an epitope recognizable by an autoantibody;
   (iii) an ACE comprising the amino acid sequence shown in SEQ ID NO: 6 or a variant thereof containing an epitope recognizable by an autoantibody;
   (iv) an ACE comprising the amino acid sequence shown in SEQ ID NO: 3 or a variant thereof containing an epitope recognizable by an autoantibody, wherein said ACE is not MST1 protein;
   (v) an ACE comprising the amino acid sequence shown in SEQ ID NO: 5 or a variant thereof containing an epitope recognizable by an autoantibody;
   (vi) an ACE comprising the amino acid sequence shown in SEQ ID NO: 1 or a variant thereof containing an epitope recognizable by an autoantibody; and
   (vii) any combination of said ACEs (i)-(vi); and
b) detecting the formation of an autoantibody-ACE complex,
wherein the detection of said autoantibody-ACE complex is indicative of the presence of said autoantibody in said subject.

The sample will generally be a biological sample susceptible of containing antibodies from a subject, and it can be obtained by conventional methods known by those of average skill in the art, depending on the nature of the sample. In a particular disclosure, said biological sample is a blood, serum or plasma sample which can be obtained by any conventional method, for example, by means of a blood extraction, etc. Blood is usually the optimal biological fluid to be used in non-invasive methods for massive screening of large populations of subjects for diagnostic purposes. On one hand, serum and plasma are easy to obtain, and on the other hand, blood circulation facilitates the contact of the blood with all the tissues of the human body, including contact with tumor tissue and its representative antigens in the case of cancer patients.

The first method disclosed herein comprises contacting a sample from a subject suspected of having CRC with an ACE selected from ACEs (i) to (vi), indicated in Table 2, and their combinations [step a)], under conditions allowing the formation of an autoantibody-ACE complex.

**Table 2**

| | ACEs |
|---|---|
| (i) | ACE comprising the amino acid sequence shown in SEQ ID NO: 4 or a variant thereof containing an epitope recognizable by an autoantibody. |
| (ii) | ACE comprising the amino acid sequence shown in SEQ ID NO: 2 or a variant thereof containing an epitope recognizable by an autoantibody. |
| (iii) | ACE comprising the amino acid sequence shown in SEQ ID NO: 6 or a variant thereof containing an epitope recognizable by an autoantibody. |
| (iv) | ACE comprising the amino acid sequence shown in SEQ ID NO: 3 or a variant thereof containing an epitope recognizable by an autoantibody; wherein said ACE is not MST1 protein. |
| (v) | ACE comprising the amino acid sequence shown in SEQ ID NO: 5 or a variant thereof containing an epitope recognizable by an autoantibody. |
| (vi) | ACE comprising the amino acid sequence shown in SEQ ID NO: 1 or a variant thereof containing an epitope recognizable by an autoantibody. |

The suitable conditions for the formation of the autoantibody-ACE complex to take place are known by the persons skilled in the art. If the sample contains autoantibodies against said ACEs, then the corresponding autoantibody-ACE complex will be formed; otherwise, said complex will not be formed.

Although said ACEs could be together in the same medium, in practice it is advantageous for said ACEs to be separated from one another. The ACEs can be in solution or suspension in a suitable medium, or can alternatively be deposited or supported on a support [e.g., a microtiter plate, beads (magnetic or non-magnetic), columns, matrices, membranes, etc.] These materials can be used in the suitable forms, such as films, sheets, plates, etc., or they can be used to coat inert carriers (e.g., paper, glass, plastic films, etc.). In a particular disclosure, the sample to be analyzed is contacted with said ACEs, separated from one another, and deposited on a suitable support.

The detection of said autoantibodies against the ACEs mentioned can be carried out by conventional methods known by those of average skill in the art. In a particular disclosure, the detection of said autoantibodies is carried out by means of immunoassay; illustrative, non-limiting examples of immunoassays known in the state of the art include immunoblot, Enzyme-linked Immunosorbent Assay (ELISA), linear immunoassay (LIA), radioimmunoassay (RIA), immunofluorescence (IF), immunohistochemistry (IHC), protein microarrays, phage microarrays, etc. The person skilled in the art will also understand that other methods based for example on electrophoretic or chromatographic techniques can be used for detecting said autoantibodies.

In a particular disclosure, the detection of autoantibodies against one or more ACEs is done by means of an ELISA. The ELISA technique is based on the premise that an immunoreagent (e.g., an antigen or an antibody) is immobilized on a solid support, and then that system is contacted with a fluid phase containing the complementary reagent which can be bound to a marker compound. There are different types of ELISA, for example, direct ELISA, indirect ELISA or sandwich ELISA.

The detection of autoantibodies against one or more ACE/ACEs by means of ELISA, for example by means of indirect ELISA, generally comprises the following steps: (a) coating a solid support with one or more ACEs, preferably separated from one another; (b) incubating the coated support of step (a) with a sample, such as a biological sample from the subject to be studied, under conditions allowing the formation of an autoantibody-ACE complex; and (c) adding a secondary antibody, which recognizes the autoantibody against the ACE/ACEs, conjugated or bound to a marker compound.

In another particular disclosure, the detection of autoantibodies against one or more ACEs is done by means of a protein microarray. A protein microarray consists of a collection of proteins immobilized on a solid support in a regular and pre-established arrangement. There are several important factors to be taken into account in the design of protein microarrays, among which, for example, the nature of the support on which the proteins (or suitable fragments thereof) are immobilized, the protein immobilization technique, the format of the microarray, the capturing agent used or the method of detection to be used are found. Different formats, supports and techniques which can be used for carrying out this first method are known in the state of the art.

The detection of autoantibodies against one or more ACEs by means of a protein microarray generally comprises the following steps: (a) coating a solid support with said ACE/ACEs, preferably separated from one another; (b) incubating the coated support of step (a) with a sample, such as a biological sample from the subject to be studied, under conditions allowing the formation of an immunocomplex of the autoantibody against the ACE/ACEs present in said sample with the corresponding antigenic determinants present in said ACEs; and (c) adding a secondary antibody, which recognizes the autoantibody against the ACE/ACEs, conjugated or bound to a marker compound.

In another particular disclosure, the detection of autoantibodies against one or more ACEs is done by means of a phage microarray. A phage microarray consists of a collection of peptides exposed on the surface of phages. In a particular disclosure, said peptides are fused to T7 phage capsid protein 10B. Said phages are immobilized on a solid support in a regular and pre-established arrangement. There are several important factors to be taken into account in the design of phage microarrays such as, for example, the nature of the support on which the phages are immobilized, the immobilization technique, the format of the microarray or the method of detection to be used. Different formats, supports and techniques that can be used for carrying out this preferred disclosureof the method disclosed herein are known in the state of the art.

The detection of autoantibodies against one or more ACE/ACEs by means of a phage microarray generally comprises the following steps: (a) coating a solid support with a phage lysate, for example, a T7 phage lysate, having a peptide or an amino acid sequence susceptible of being recognized by an autoantibody exposed on the phage surface; (b) incubating the coated support of step (a) with a sample, such as a biological sample from the subject to be studied, under conditions allowing the formation of an autoantibody-ACE complex; and (c) adding a secondary antibody, which recognizes the autoantibody against the ACE/ACEs, conjugated or bound to a marker compound. In a specific disclosure, said phage microarray comprises a phage selected from the group consisting of:
(i) a phage comprising the amino acid sequence shown in SEQ ID NO: 4 or a variant thereof containing an epitope recognizable by an autoantibody, wherein said amino acid sequence is exposed on the phage surface;
(ii) a phage comprising the amino acid sequence shown in SEQ ID NO: 2 or a variant thereof containing an epitope recognizable by an autoantibody, wherein said amino acid sequence is exposed on the phage surface;
(iii)a phage comprising the amino acid sequence shown in SEQ ID NO: 6 or a variant thereof containing an epitope recognizable by an autoantibody, wherein said amino acid sequence is exposed on the phage surface;
(iv) a phage comprising the amino acid sequence shown in SEQ ID NO: 3 or a variant thereof containing an epitope recognizable by an autoantibody, wherein said amino acid sequence is exposed on the phage surface;
(v) a phage comprising the amino acid sequence shown in SEQ ID NO: 5 or a variant thereof containing an epitope recognizable by an autoantibody, wherein said amino acid sequence is exposed on the phage surface;
(vi) a phage comprising the amino acid sequence shown in SEQ ID NO: 1 or a variant thereof containing an epitope recognizable by an autoantibody, wherein said amino acid sequence is exposed on the phage surface; and
(vii) any of the combinations of (i) to (vi).

The marker bound to the secondary antibody referred to in these techniques is a compound capable of giving rise to a chromogenic, fluorogenic, radioactive and/or chemiluminescent signal which allows the detection, identification and, optionally, quantification of the amount of the autoantibody versus the ACE/ACEs present in the analyzed sample. In a particular disclosure, said marker compound is selected from the group consisting of radioisotopes, enzymes, fluorophores or any molecule susceptible of being conjugated with another molecule or detected and/or quantified directly. This marker compound can bind to the autoantibody directly, or through another compound. Illustrative non-limiting examples of said marker compounds binding directly to the autoantibody include enzymes, such as alkaline phosphatase, peroxidase, etc., radioactive isotopes, such as ³²P, ³⁵S, etc., fluorochromes, such as fluorescein, etc., or metal particles, for their direct detection by means of colorimetry, auto-radiography, fluorometry, or metallography, respectively.

The detection of the autoantibodies can be carried out by applying a single technique or it can be carried out by applying a combination of two or more techniques; by way of illustration, some autoantibodies can be detected by means of an ELISA and others by means of a protein microarray, or some by means of an ELISA and others by means of a phage microarray, or some by means of a protein microarray and others by means of a phage microarray, etc.

In a particular disclosure, the sample to be analyzed is contacted with a single ACE selected from the group of ACEs (i)-(vi) shown in Table 2, and their combinations, under conditions allowing the formation of an autoantibody-ACE complex for the purpose of identifying autoantibodies against said ACE. In another particular disclosure, said biological sample is contacted with two or more of said ACEs susceptible of being recognized by said autoantibodies, separated from one another, optionally deposited on a suitable support, for the purpose of identifying autoantibodies against said ACEs.

In a particular disclosure, the first method disclosed herein comprises the detection of an autoantibody against an ACE comprising the amino acid sequence shown in SEQ ID NO: 4 or a variant thereof containing an epitope recognizable by an autoantibody, and furthermore the detection of an autoantibody selected from the group consisting of: (i) an autoantibody against an ACE comprising the amino acid sequence shown in SEQ ID NO: 2 or a variant thereof containing an epitope recognizable by an autoantibody; (ii) an autoantibody against an ACE comprising the amino acid sequence shown in SEQ ID NO: 6 or a variant thereof containing an epitope recognizable by an autoantibody; (iii) an autoantibody against an ACE comprising the amino acid sequence shown in SEQ ID NO: 3 or a variant thereof containing an epitope recognizable by an autoantibody; (iv) an autoantibody against an ACE comprising the amino acid sequence shown in SEQ ID NO: 5 or a variant thereof containing an epitope recognizable by an autoantibody; (v) an autoantibody against an ACE comprising the amino acid sequence shown in SEQ ID NO: 1 or a variant thereof containing an epitope recognizable by an autoantibody; and (vi) any combination of autoantibodies (i) to (v). In a more specific form of said particular disclosure, said first method of the disclosure comprises the detection of an autoantibody against an ACE comprising the amino acid sequence shown in SEQ ID NO: 4 or a variant thereof containing an epitope recognizable by an autoantibody, and furthermore the detection of any 1, 2, 3, 4, or 5 of said autoantibodies (i) to (v) previously indicated.

In a specific disclosure, said ACE is SULF1 protein or a variant or fragment thereof containing an epitope recognizable by an autoantibody, whereas in another specific disclosure, said ACE is a phage comprising the amino acid sequence shown in SEQ ID NO: 4 or a variant thereof containing an epitope recognizable by an autoantibody, wherein said amino acid sequence is exposed on the phage surface.

In another particular disclosure, the first method of the disclosurecomprises the detection of an autoantibody against an ACE comprising the amino acid sequence shown in SEQ ID NO: 1 or a variant thereof containing an epitope recognizable by an autoantibody, and furthermore the detection of an autoantibody selected from the group consisting of: (i') an autoantibody against an ACE comprising the amino acid sequence shown in SEQ ID NO: 4 or a variant thereof containing an epitope recognizable by an autoantibody; (ii') an autoantibody against an ACE comprising the amino acid sequence shown in SEQ ID NO: 2 or a variant thereof containing an epitope recognizable by an autoantibody; (iii') an autoantibody against an ACE comprising the amino acid sequence shown in SEQ ID NO: 6 or a variant thereof containing an epitope recognizable by an autoantibody; (iv') an autoantibody against an ACE comprising the amino acid sequence shown in SEQ ID NO: 3 or a variant thereof containing an epitope recognizable by an autoantibody; (v') an autoantibody against an ACE comprising the amino acid sequence shown in SEQ ID NO: 5 or a variant thereof containing an epitope recognizable by an autoantibody; and (vi') any combination of autoantibodies (i') to (v'). In a more specific form, said first method of the disclosurecomprises the detection of an autoantibody against an ACE comprising the amino acid sequence shown in SEQ ID NO: 1 or a variant thereof containing an epitope recognizable by an autoantibody, and furthermore the detection of any 1, 2, 3, 4, or 5 of said autoantibodies (i') to (v') previously indicated.

Step b) of the method for detecting autoantibodies of the disclosurecomprises detecting the formation of an autoantibody-ACE complex. This step can be carried out by conventional methods known by those of average skill in the art, for the detection of the formation of antibody-antigen complexes (in this case, autoantibody-ACE).

In a particular disclosure, by way of non-limiting illustration, for the detection of said complex, a conjugate comprising an antibody recognizing the autoantibody and a marker (labeled secondary antibody) can be added under conditions allowing the formation of an (autoantibody-ACE)-antibody/marker complex and detecting the formation of said complex. If the biological sample contains autoantibodies against one or more of said ACEs, then the autoantibody-ACE complex will have been previously formed, whereby when said complex is contacted with said conjugate comprising the antibody and the marker in suitable conditions, (autoantibody-ACE)-antibody/marker complex is formed, which will be viewed by means of the suitable technique depending on the marker used, as mentioned below; whereas, otherwise, i.e., when the biological sample does not contain autoantibodies against said ACE/ACEs then said (autoantibody-ACE)-antibody/marker complex will not be formed. The suitable conditions for the formation of this latter complex to take place are known by the persons skilled in the art.

Virtually any indicator reagent which allows detecting said (autoantibody-ACE)-antibody/marker complex can be used in putting the present disclosureinto practice. By way of non-limiting illustration, said marker can be an enzyme catalyzing a detectable reaction (e.g., peroxidase, glycosidase, alkaline phosphatase, glucose-6-phosphate dehydrogenase, β-galactosidase, β-glucosidase, β-glucuronidase, etc.), a compound generating a signal when it forms part of said complex (e.g., a fluorescent compound or fluorophore, such as fluorescein, rhodamine, etc.; a (chemi)luminescent compound, such as a dioxetane, an acridinium, a phenanthridinium, ruthenium, luminol, etc.), a radioactive element (e.g., sulfur, iodine, etc.), etc. In a particular disclosure, said marker is a peroxidase. The selection of a particular marker is not critical, provided that it is capable of producing a signal by itself or together with one or more additional substances. The (autoantibody-ACE)-antibody/marker complex formed can thus be detected or displayed by any suitable technique, depending on the chosen marker, known by those of average skill in the art, using the suitable devices, for example, by means of techniques based on colorimetric, fluorometric, (chemi)luminescent, radioactive methods, etc., all of them known by those of average skill in the art.

The conjugate comprising said antibody which recognizes said autoantibody and said marker can be obtained by conventional methods known by those of average skill in the art.

By way of illustration, when the marker is an enzyme, the detection of the complex in question can be carried out by contacting said complex with a suitable substrate and, optionally, with suitable enzymatic amplification agents and/or activators. Illustrative non-limiting examples of said substrates include:
- For alkaline phosphatase:
   Chromogenic: substrates based on p-nitrophenyl phosphate (p-NPP), 5-bromo-4-chloro-3-indolyl phosphate/nitroblue tetrazolium (BCIP/NPT), etc.
   Fluorogenic: 4-methylumbeliphenyl phosphate (4-MUP), 2-(5'chloro-2'-phosphoryloxyphenyl)-6-chloro-4-(3H)-quinazolinone (CPPCQ), 3,6-fluorescein-diphosphate (3,6-FDP), etc.
- For peroxidases:
   Chromogenic: substrates based on 2,2-azinobis(3-ethylbenzothiazoline-6-sulfonic) (ABTS) acid, o-phenylenediamine (OPT), 3,3',5,5'-tetramethylbenzidine (TMB), o-dianisidine, 5-aminosalicylic acid, 3-dimethylaminobenzoic (DMAB) acid and 3-methyl-2-benzothiazolinehydrazone (MBTH), 3-amino-9-ethylcarbazole (AEC) and 3,3'-diaminobenzidine (DAB) tetrachloride, etc.
   Fluorogenic: 4-hydroxy-3-methoxyphenylacetic acid, reduced phenoxazines and reduced benzothiazines, including the reagent Amplex® Red, Amplex UltraRed, reduced dihydroxanthenes, etc.
- For glycosidases:
   Chromogenic: substrates based on o-nitrophenyl-β-D-galactoside (o-NPG), p-nitrophenyl-β-D-galactoside and 4-methylumbeliphenyl-β-D-galactoside (MUG) for β-D-galactosidase, etc.
   Fluorogenic: resorufin β-D-galactopyranoside, fluorescein digalactoside (FDG), fluorescein diglucuronide, 4-methylumbelliferyl beta-D-galactopyranoside, carboxyumbelliferyl beta-D-galactopyranoside, fluorinated coumarin beta-D-galactopyranosides, etc.

By means of putting the first method of the disclosureinto practice, it is possible to detect and obtain autoantibodies against the ACEs indicated in Table 2. Additionally, the level or amount of said autoantibodies against said ACEs present in the sample under study could be determined (quantified) if desired because the signal generated by some markers (e.g., enzymes, etc.) is proportional to the amount of autoantibody present in said sample.

Optionally, if desired, the autoantibody-ACE complex can be isolated by means of conventional techniques, for example, by means of using immunoprecipitation techniques, etc., and the sequence of the autoantibody binding to the ACE can be subsequently sequenced by means of conventional proteomic methods described in the art, such as the determination of the peptide fingerprint or MS/MS analysis (Vikas Dhingraa, et al. 2005. International Journal of Pharmaceutics 299 (1-2):1-18; Hanash SM et al. Nature. 2008 Apr 3;452(7187):571-9).

According to the first method of the disclosure, the detection of the autoantibody-ACE complex is indicative of the presence of the corresponding specific autoantibody (or autoantibodies) against said ACE/ACEs in the analyzed sample and, therefore, in the analyzed subject.

In a particular disclosure, the formation of said autoantibody-ACE complex in said sample can be correlated with a diagnosis of CRC in the subject the analyzed sample is from, or with the prognosis of said disease, or with tracking said disease progression. In the sense used in this description, the term "correlate" refers to comparing the presence or amount of the indicator in a subject (e.g., a subject suspected of having CRC) with its presence or amount in subjects having said disease (CRC), or predisposed to develop it, or in subjects free of said disease.

As used herein, the term "diagnosis" generally refers to the process whereby a disease, nosological entity, syndrome, or any disease-health condition is identified. Particularly, the term "diagnosis of colorectal cancer (or CRC)" refers to the capacity to identify or detect the presence of CRC; this detection, as it is understood by a person skilled in the art, does not claim to be correct in 100% of the analyzed samples. However, it requires that a statistically significant amount of the analyzed samples are classified correctly. The amount that is statistically significant can be established by a person skilled in the art by using different statistical tools; illustrative, non-limiting examples of said statistical tools include determining confidence intervals, determining the p-value, the Student's t-test or Fisher's discriminant functions, etc. (see, for example, Dowdy and Wearden, Statistics for Research, John Wiley & Sons, New York 1983). The confidence intervals are preferably at least 90%, at least 95%, at least 97%, at least 98% or at least 99%. The p-value is preferably less than 0.1, less than 0.05, less than 0.01, less than 0.005 or less than 0.0001. The teachings of the present disclosure advantageously allow correctly detecting CRC in at least 50%, preferably in at least 60%, more preferably in at least 70%, even more preferably in at least 80%, or still even more preferably in at least 90% of the subjects of a specific group or population analyzed.

In another particular disclosure, the first method disclosed herein comprises comparing the immunoreactivity of the analyzed sample with the immunoreactivity of a second sample from the same subject in a later time period. It is therefore possible to either evaluate disease progression or to evaluate the efficacy of the treatment if said second sample has been obtained after the subject has been treated for CRC. In the sense used in this description, the term "immunoreactivity" refers to the presence or level of binding of an antibody or antibodies in a sample to one or more target antigens, for example, the ACEs of Table 2. An "immunoreactivity pattern" refers to a binding profile of antibodies in a sample (autoantibodies) to a plurality of target antigens (e.g., the ACEs of Table 2).

In another disclosure, the first method disclosed herein further comprises analyzing the presence of one or more additional markers of CRC, for example, CEA or autoantibodies against Pim1, SRC, MAPKAPK3, FGFR4, STK4 and/or ACVR2B proteins.

### Method for detecting autoantibodies

It is also disclosed a method for detecting an autoantibody in a sample, comprising:
(a) contacting a sample with an antibody capturing entity (ACE), wherein said ACE is selected from the group consisting of:
   (i) an ACE comprising the amino acid sequence shown in SEQ ID NO: 4 or a variant thereof containing an epitope recognizable by an autoantibody;
   (ii) an ACE comprising the amino acid sequence shown in SEQ ID NO: 2 or a variant thereof containing an epitope recognizable by an autoantibody;
   (iii) an ACE comprising the amino acid sequence shown in SEQ ID NO: 6 or a variant thereof containing an epitope recognizable by an autoantibody;
   (iv) an ACE comprising the amino acid sequence shown in SEQ ID NO: 3 or a variant thereof containing an epitope recognizable by an autoantibody, wherein said ACE is not MST1 protein;
   (v) an ACE comprising the amino acid sequence shown in SEQ ID NO: 5 or a variant thereof containing an epitope recognizable by an autoantibody;
   (vi) an ACE comprising the amino acid sequence shown in SEQ ID NO: 1 or a variant thereof containing an epitope recognizable by an autoantibody; and
   (vii) any combination of said ACEs (i)-(vi); and
(b)detecting the formation of an autoantibody-ACE complex, wherein the detection of said autoantibody-ACE complex is indicative of the presence of said autoantibody in said sample. The invention relates to a method for detecting an autoantibody in a sample, method of the invention, comprising:
   a) contacting a sample from a subject with an antibody capturing entity (ACE), wherein said ACE is an ACE comprising the amino acid sequence shown in SEQ ID NO: 4 or a peptide or protein having at least 90% sequence identity with respect to SEQ ID NO: 4 and that is recognized by an autoantibody against SEQ ID NO: 4; and
   b) detecting the formation of a complex between an autoantibody and said ACE,
   wherein the detection of said autoantibody-ACE complex is indicative of the presence of said autoantibody in said sample, wherein the sample is blood, plasma or serum.

Generally, the characteristics of said ACE referred to in the method of the invention are the same as the characteristics of the ACE referred to in the first method of the disclosure. The techniques for detecting the autoantibodies according to the method of the invention are the same as those mentioned in relation to the first method of the disclosure, so they are herein incorporated by reference.

The particular disclosures of the first method disclosed herein, as well as the definitions of the terms used also apply to the method of the invention, so they are incorporated in this method of the invention by reference.

This method of the invention allows correlating the results obtained with those pathologies in which immune responses with the subsequent production of autoantibodies are generated. Illustrative, non-limiting examples of said pathologies include some types of cancer, e.g., CRC, hepatocellular carcinoma (Imai, H et al. Intervirology 35:73-85), breast cancer, prostate cancer (Wang X et al. N Engl J Med. 2005; 353(12) :1224-35), lung cancer, etc. and autoimmune diseases.

In a particular embodiment of the method of the invention, the sample is a sample from a subject suspected of having colorectal cancer (CRC). More particularly the method of the invention further comprises correlating the formation of the autoantibody-ACE complex in the sample from the subject with a diagnosis of CRC or further comprises comparing the immunoreactivity of the sample with the immunoreactivity of a second sample from the same subject in a later time period. Particularly said second sample from the subject has been obtained after said subject has been treated for CRC.

In a particular embodiment, the method of the invention comprises the detection of an autoantibody against an ACE comprising the amino acid sequence shown in SEQ ID NO: 4 or a protein or peptide having at least 90% sequence identity with respect to SEQ ID NO: 4 containing an epitope recognizable by an autoantibody against SEQ ID NO: 4,, and furthermore the detection of an autoantibody selected from the group consisting of:
(i) an autoantibody against an ACE comprising the amino acid sequence shown in SEQ ID NO: 2 or a protein or peptide having at least 90% sequence identity with respect to SEQ ID NO: 2 containing an epitope recognizable by an autoantibody against SEQ ID NO: 2;
(ii) an autoantibody against an ACE comprising the amino acid sequence shown in SEQ ID NO: 6 or a protein or peptide having at least 90% sequence identity with respect to SEQ ID NO: 6 thereof containing an epitope recognizable by an autoantibody against SEQ ID NO: 6;
(iii)an autoantibody against an ACE comprising the amino acid sequence shown in SEQ ID NO: 3 or a protein or peptide having at least 90% sequence identity with respect to SEQ ID NO: 3 thereof containing an epitope recognizable by an autoantibody against SEQ ID NO: 3;
(iv) an autoantibody against an ACE comprising the amino acid sequence shown in SEQ ID NO: 5 or a protein or peptide having at least 90% sequence identity with respect to SEQ ID NO: 5 thereof containing an epitope recognizable by an autoantibody against SEQ ID NO: 5;
(v) an autoantibody against an ACE comprising the amino acid sequence shown in SEQ ID NO: 1 or a protein or peptide having at least 90% sequence identity with respect to SEQ ID NO: 1 thereof containing an epitope recognizable by an autoantibody against SEQ ID NO: 1; and (vi) any combination of autoantibodies (i) to (v).

In another preferred embodiment of the method of the invention, said ACE is sulfatase 1 (SULF1) or a protein or peptide having at least 90% sequence identity with respect to SULF1 containing an epitope recognizable by an autoantibody against SULF1,
or alternatively
said ACE is a phage comprising the amino acid sequence shown in SEQ ID NO: 4 or a protein or peptide having at least 90% sequence identity with respect to SEQ ID NO: 4 containing an epitope recognizable by an autoantibody against SEQ ID NO: 4, wherein said amino acid sequence is exposed on the phage surface.

It is discloseda method comprising the detection of an autoantibody against an ACE comprising the amino acid sequence shown in SEQ ID NO: 1 or a variant thereof containing an epitope recognizable by an autoantibody, and furthermore the detection of an autoantibody selected from the group consisting of:
(i) an autoantibody against an ACE comprising the amino acid sequence shown in SEQ ID NO: 4 or a variant thereof containing an epitope recognizable by an autoantibody;
(ii) an autoantibody against an ACE comprising the amino acid sequence shown in SEQ ID NO: 2 or a variant thereof containing an epitope recognizable by an autoantibody;
(iii) an autoantibody against an ACE comprising the amino acid sequence shown in SEQ ID NO: 6 or a variant thereof containing an epitope recognizable by an autoantibody;
(iv) an autoantibody against an ACE comprising the amino acid sequence shown in SEQ ID NO: 3 or a variant thereof containing an epitope recognizable by an autoantibody;
(v) an autoantibody against an ACE comprising the amino acid sequence shown in SEQ ID NO: 5 or a variant thereof containing an epitope recognizable by an autoantibody; and
(vi) any combination of autoantibodies (i) to (v).

### Antibody capturing entity (ACE)

It is also disclosed an antibody capturing entity (ACE) selected from the group consisting of:
(i) an ACE comprising the amino acid sequence shown in SEQ ID NO: 4 or a variant thereof containing an epitope recognizable by an autoantibody, wherein said ACE is not SULF1 protein;
(ii) an ACE comprising the amino acid sequence shown in SEQ ID NO: 2 or a variant thereof containing an epitope recognizable by an autoantibody, wherein said ACE is not GRN protein;
(iii) an ACE comprising the amino acid sequence shown in SEQ ID NO: 6 or a variant thereof containing an epitope recognizable by an autoantibody, wherein said ACE is not GTF2i protein; and
(iv) an ACE comprising the amino acid sequence shown in SEQ ID NO: 3 or a variant thereof containing an epitope recognizable by an autoantibody, wherein said ACE is not MST1 protein;
(v) an ACE comprising the amino acid sequence shown in SEQ ID NO: 5 or a variant thereof containing an epitope recognizable by an autoantibody, wherein said ACE is not SREBF2 protein;
(vi) an ACE comprising the amino acid sequence shown in SEQ ID NO: 1 or a variant thereof containing an epitope recognizable by an autoantibody, wherein said ACE is not NHSL1 protein; and
(vii) any combination of ACEs (i) to (vi).

As previously defined, an ACE is a macromolecular entity, for example, a peptide, a protein or a phage, binding specifically to an antibody (or autoantibody). In a particular disclosure, said ACE comprises a peptide or a protein binding specifically to an antibody (or autoantibody). Said peptide can either be immobilized on a support or exposed on the phage surface. In a preferred particular disclosure, said ACE is a peptide, a protein or a phage on the surface of which said peptide or said protein is exposed. Said ACE can be immobilized on a solid support if desired.

The invention relates to an antibody capturing entity (ACE) comprising the amino acid sequence shown in SEQ ID NO: 4, wherein said ACE is not sulfatase-1 (SULF1) protein. In a preferred embodiment of the antibody capturing entity (ACE) according to the invention, said ACE is a phage comprising the amino acid sequence shown in SEQ ID NO: 4, wherein said amino acid sequence is exposed on the phage surface.

In a particular disclosure, the ACE is selected from the group consisting of:
(i) a phage comprising the amino acid sequence shown in SEQ ID NO: 1 or a variant thereof containing an epitope recognizable by an autoantibody, wherein said amino acid sequence is exposed on the phage surface;
(ii) a phage comprising the amino acid sequence shown in SEQ ID NO: 2 or a variant thereof containing an epitope recognizable by an autoantibody, wherein said amino acid sequence is exposed on the phage surface;
(iii) a phage comprising the amino acid sequence shown in SEQ ID NO: 3 or a variant thereof containing an epitope recognizable by an autoantibody, wherein said amino acid sequence is exposed on the phage surface;
(iv) a phage comprising the amino acid sequence shown in SEQ ID NO: 4 or a variant thereof containing an epitope recognizable by an autoantibody, wherein said amino acid sequence is exposed on the phage surface;
(v) a phage comprising the amino acid sequence shown in SEQ ID NO: 5 or a variant thereof containing an epitope recognizable by an autoantibody, wherein said amino acid sequence is exposed on the phage surface;
(vi) a phage comprising the amino acid sequence shown in SEQ ID NO: 6 or a variant thereof containing an epitope recognizable by an autoantibody, wherein said amino acid sequence is exposed on the phage surface; and
(vii) any of the combinations of (i) to (vi).

Said phages can be obtained by conventional methods known by those of average skill in the art, and more specifically by means of the process described in Example 1.

The ACEs disclosed herein can be used in the detection of antibodies or autoantibodies against said ACEs in a sample, particularly against the amino acid sequences identified as SEQ ID NOs: 1-6 present in said ACEs, and the presence of said autoantibodies in said sample can be correlated with the diagnosis, prognosis, monitoring the progression, or efficacy of the treatment, of a disease, so that said autoantibodies are markers, for example, of CRC.

### Composition of the invention

It is also disclosed composition 1 of the disclosure, comprising an ACE disclosed in the document. As previously indicated, in a particular disclosure, said ACE can be a peptide, a protein or a phage.

In a particular disclosure, composition 1 of the disclosurecomprises at least one ACE disclosed herein. In another particular disclosure, composition 1 of the disclosurecomprises at least 2 ACEs of the disclosure, for example, 2, 3, 4, 5 or even the 6 ACEs of the disclosure.

In another particular disclosure, composition 1 comprises an ACE comprising the amino acid sequence shown in SEQ ID NO: 4 or a variant thereof containing an epitope recognizable by an autoantibody, and furthermore at least one ACE selected from the group consisting of:
(i) an ACE comprising the amino acid sequence shown in SEQ ID NO: 2 or a variant thereof containing an epitope recognizable by an autoantibody;
(ii) an ACE comprising the amino acid sequence shown in SEQ ID NO: 6 or a variant thereof containing an epitope recognizable by an autoantibody;
(iii) an ACE comprising the amino acid sequence shown in SEQ ID NO: 3 or a variant thereof containing an epitope recognizable by an autoantibody;
(iv) an ACE comprising the amino acid sequence shown in SEQ ID NO: 5 or a variant thereof containing an epitope recognizable by an autoantibody;
(v) an ACE comprising the amino acid sequence shown in SEQ ID NO: 1 or a variant thereof containing an epitope recognizable by an autoantibody; and
(vi) any combination of ACEs (i) to (v).

In another particular disclosure, composition 1 of the disclosure comprises an ACE comprising the amino acid sequence shown in SEQ ID NO: 1 or a variant thereof containing an epitope recognizable by an autoantibody, and furthermore at least one ACE selected from the group consisting of:
(i) an ACE comprising the amino acid sequence shown in SEQ ID NO: 2 or a variant thereof containing an epitope recognizable by an autoantibody;
(ii) an ACE comprising the amino acid sequence shown in SEQ ID NO: 3 or a variant thereof containing an epitope recognizable by an autoantibody;
(iii) an ACE comprising the amino acid sequence shown in SEQ ID NO: 4 or a variant thereof containing an epitope recognizable by an autoantibody;
(iv) an ACE comprising the amino acid sequence shown in SEQ ID NO: 5 or a variant thereof containing an epitope recognizable by an autoantibody;
(v) an ACE comprising the amino acid sequence shown in SEQ ID NO: 6 a variant thereof containing an epitope recognizable by an autoantibody; and
(vi) any combination of ACEs (i) to (v).

In another particular disclosure, composition 1 of the disclosure comprises at least one ACE disclosed in the present document and at least one protein selected from the group consisting of SULF1 protein or a variant thereof, MST1 protein or a variant thereof, and their combinations. The person skilled in the art will note that it is possible to use SULF1 or MST1 proteins of different species; nevertheless, in a preferred disclosure, composition 1 of the disclosureincludes SULF1 or MST1 proteins of a human origin, such as human SULF1 protein, with NCBI database accession number (May 1, 2011 version) EAW86954.1 and its amino acid sequence is SEQ ID NO: 10, and human MST1 protein with NCBI database accession number (May 1, 2011 version) AAA83254.1 and its amino acid sequence is SEQ ID NO: 9. The term "variant" has already been defined above in the "Definitions" section.

In another particular disclosure, composition 1 of the disclosurecomprises:
a) an ACE selected from the group consisting of:
   (i) an ACE comprising the amino acid sequence shown in SEQ ID NO: 4 or a variant thereof containing an epitope recognizable by an autoantibody,
   (ii) an ACE comprising the amino acid sequence shown in SEQ ID NO: 2 or a variant thereof containing an epitope recognizable by an autoantibody;
   (iii)an ACE comprising the amino acid sequence shown in SEQ ID NO: 6 or a variant thereof containing an epitope recognizable by an autoantibody;
   (iv) an ACE comprising the amino acid sequence shown in SEQ ID NO: 3 or a variant thereof containing an epitope recognizable by an autoantibody;
   (v) an ACE comprising the amino acid sequence shown in SEQ ID NO: 5 or a variant thereof containing an epitope recognizable by an autoantibody;
   (vi) an ACE comprising the amino acid sequence shown in SEQ ID NO: 1 or a variant thereof containing an epitope recognizable by an autoantibody; and
   (vii)any combination of ACEs (i) to (vi); and
b) a protein selected from the group consisting of SULF1 protein or a variant thereof, MST1 protein or a variant thereof, and their combinations.

In a preferred disclosure, said composition 1 of the disclosurecomprises:
a) an ACE selected from the group consisting of:
   (i) an ACE comprising the amino acid sequence shown in SEQ ID NO: 2 or a variant thereof containing an epitope recognizable by an autoantibody,
   (ii) an ACE comprising the amino acid sequence shown in SEQ ID NO: 6 or a variant thereof containing an epitope recognizable by an autoantibody;
   (iii)an ACE comprising the amino acid sequence shown in SEQ ID NO: 5 or a variant thereof containing an epitope recognizable by an autoantibody;
   (iv) an ACE comprising the amino acid sequence shown in SEQ ID NO: 1 or a variant thereof containing an epitope recognizable by an autoantibody; and
   (v) any combination of ACEs (i) to (iv); and
b) a protein selected from the group consisting of SULF1 protein or a variant thereof, MST1 protein or a variant thereof, and their combinations.

The composition of the invention comprises an antibody capturing entity (ACE) according to the invention, composition 1 of the invention.The composition 1 of the disclosurehas provided good results in the diagnosis of CRC [Example 4].

In a preferred embodiment, the composition of the invention further comprises at least one ACE according to the invention, and at least one protein selected from the group consisting of sulfatase 1 (SULF1) protein or a protein or peptide having at least 90% sequence identity with respect to SULF1 and recognizable by an autoantibody against SULF1, MST1 protein or a protein or peptide having at least 90% sequence identity with respect to MST1 and recognizable by an autoantibody against MST1, and their combinations.

It is also disclosed composition 2 of the disclosure, comprising SULF1 protein or a variant thereof and MST1 protein or a variant thereof. The characteristics of said SULF1 and MST1 proteins, and of their variants, have been mentioned previously.

In a particular disclosure, composition 2 of the disclosurefurther comprises at least one ACE of the disclosure. In another particular disclosure, composition 2 of the disclosurecomprises at least 2 ACEs of the disclosure.

In another particular disclosure, said composition 2 of the disclosurecomprises:
a) SULF1 protein or a variant thereof;
b) MST1 protein or a variant thereof; and
c) an ACE selected from the group consisting of:
   (i) an ACE comprising the amino acid sequence shown in SEQ ID NO: 2 or a variant thereof containing an epitope recognizable by an autoantibody,
   (ii) an ACE comprising the amino acid sequence shown in SEQ ID NO: 6 or a variant thereof containing an epitope recognizable by an autoantibody;
   (iii)an ACE comprising the amino acid sequence shown in SEQ ID NO: 5 or a variant thereof containing an epitope recognizable by an autoantibody;
   (iv) an ACE comprising the amino acid sequence shown in SEQ ID NO: 1 or a variant thereof containing an epitope recognizable by an autoantibody; and
   (v) any combination of ACEs (i) to (iv).

The invention also relates to a composition, composition 2 of the invention, comprising sulfatase 1(SULF1) protein or a protein or peptide having at least 90% sequence identity with respect to SULF1 and recognizable by an autoantibody against SULF1 and MST1 protein or a protein or peptide having at least 90% sequence identity with respect to MST1 and recognizable by an autoantibody against MST1 and an antibody capturing entity (ACE) according to the invention.

In a particular embodiment, both composition 1 of the invention and composition 2 of the invention are supported on a solid support.

### Kit of the invention and applications

In another aspect, the invention relates to a kit, hereinafter kit 1 of the invention, comprising a composition of the invention. In a particular embodiment, said composition of the invention is composition 1 of the invention. In another particular embodiment, said composition of the invention is composition 2 of the invention.

In another aspect, the invention relates to the *in vitro* use of kit 1 of the invention for:
- detecting an antibody in a sample,
- detecting an autoantibody in a subject suspected of having colorectal cancer (CRC),
- diagnosing whether a subject has CRC,
- determining the risk of a subject developing CRC,
- monitoring CRC progression in a subject,
- evaluating the efficacy of a treatment against CRC, or
- predicting survival of a subject who has CRC.

For said applications, kit 1 of the invention will include the reagents necessary for detecting autoantibodies against at least one ACE of Table 2.

In a particular embodiment, kit 1 of the invention comprises an ACE of the invention. In another particular embodiment, the kit of the invention comprises composition 1 of the invention. In another particular embodiment, the kit of the invention comprises composition 2 of the invention. It is disclosed a kit 1 comprising a protein selected from the group consisting of SULF1 protein or a variant thereof, MST1 protein or a variant thereof, and their combinations.

In another aspect, the invention relates to a kit, hereinafter kit 2 of the invention, comprising a reagent for detecting SULF1 protein or a protein or peptide having at least 90% sequence identity with respect to SULF1 and recognizable by an autoantibody against SULF1. In a particular embodiment, said kit 2 of the invention further comprises a reagent for detecting MST1 protein or a protein or peptide having at least 90% sequence identity with respect to MST1 and recognizable by an autoantibody against MST1. In another particular embodiment, kit 2 of the invention comprises an ACE selected from the group of ACEs mentioned in Table 2.

Kit 2 of the invention can be used in the same applications as kit 1 of the invention.

Both kit 1 of the invention and kit 2 of the invention can further contain all those reagents necessary for detecting the amount of autoantibodies against the ACEs defined previously, or against the SULF1, MST1 proteins or their variants, such as but not being limited to the following for example
- secondary antibodies labeled with a marker specifically recognizing the autoantibody-ACE complexes;
- substrates for the markers present in said labeled secondary antibodies; and
- positive and/or negative controls.

Likewise, said kits 1 and 2 of the invention can further include, without any type of limitation, buffers, agents for preventing contamination, protein degradation inhibitors, etc. In addition, the kit of the invention can include all the supports and containers necessary for being put into practice and for optimization. Preferably, the kit further comprises instructions for use.

### Method for the diagnosis of CRC

The authors of the present invention have additionally found that the overexpression of SULF1 protein is correlated with CRC, as shown in Figure 3.

Therefore, it is also disclosed a method for diagnosing whether a subject suffers colorectal cancer (CRC), comprising determining SULF1 protein level in a sample from said subject, wherein if said SULF1 protein level is greater than the SULF1 protein level of a reference sample, it is indicative of the subject having CRC.

For putting this method disclosed herein into practice, the sample can preferably be a tissue sample, such as a colon or tumor tissue sample.

The term "diagnosis" has already been defined above.

The methods for determining protein level (concentration) are well-known by a person skilled in the art and include a number of alternatives. Virtually any method which allows determining (quantifying) SULF1 protein level can be used in putting the method disclosed herein into practice.

In a particular disclosure, SULF1 protein level is quantified by means of a conventional method allowing detecting and quantifying said protein in a sample to be studied, such as a sample from a subject. By way of non-limiting illustration, said SULF1 protein level can be determined by means of an immunoassay, for example, ELISA, etc., by means of nuclear magnetic resonance (NMR) or by means of any other suitable technique known in the state of the art. In a preferred disclosure, protein level is determined by means of an immunoassay. In a preferred particular disclosure, said immunoassay is an immunoblot (Western blot or membrane immunodetection). To that end, briefly, a protein extract is obtained from a biological sample isolated from a subject and the protein is separated by electrophoresis in a support medium capable of retaining it. Once the proteins are separated, they are transferred to a different support or membrane where they can be detected by using specific antibodies recognizing the protein in question (SULF1). Said membrane is hybridized with a first specific antibody (or primary antibody) recognizing SULF1 protein. Then the membrane is hybridized with a second antibody (or secondary antibody) specifically recognizing the primary antibody and conjugated or bound with a marker compound. In an alternative disclosure, the antibody recognizing a SULF1 protein is conjugated or bound to a marker compound, and the use of a secondary antibody is not necessary. Different formats, supports and techniques that can be used for performing this preferred disclosure of the method disclosed herein are known.

In another preferred particular disclosure, the immunoassay comprises an immunohistochemical assay. Immunohistochemistry techniques allow the identification of characteristic antigenic determinants in tissue and cytology samples. Analysis by means of immunohistochemistry (IHC) is performed on tissue sections, either frozen or included in paraffin, from a biological sample isolated from a subject. These sections are hybridized with a specific antibody or primary antibody recognizing specific antibodies recognizing a SULF1 protein. The sections are then hybridized with a secondary antibody capable of specifically recognizing the primary antibody and is conjugated or bound to a marker compound. In an alternative disclosure, the antibody recognizing SULF1 protein is conjugated or bound to a marker compound, and the use of a secondary antibody is not necessary.

By way of non-limiting illustration, "SULF1 protein level" refers but is not limited to a quantifiable, semiquantifiable, or relative amount of said SULF1 protein, as well as to any other value or parameter related to said protein or which can be derived therefrom. Said values or parameters comprise signal intensity values obtained from any of the physical or chemical properties of said protein obtained either by means of direct measurement, e.g., intensity values of mass spectroscopy, nuclear magnetic resonance, etc., or by means of indirect measurement, e.g., by means of any of the systems of measurement described herein, for example, by means of the measurement obtained from a secondary component or a biological measurement system (e.g., the measurement of cell responses, ligands, "tags" or enzymatic reaction products). The SULF1 protein level determined in a sample, such as a biological sample from the subject subjected to study, is said to be "greater" than the reference level of said SULF1 protein when, according to the disclosure, the level of said protein in the biological sample to be analyzed is at least 1.5 times, 5 times, 10 times, 20 times, 30 times, 40 times, 50 times, 60 times, 70 times, 80 times, 90 times, 100 times or even more, with respect to the reference level of said protein.

As used herein, the term "reference level" generally refers to the level of a product, for example, SULF1 protein, present in control subjects. In a particular disclosure, said control subjects are subjects who do not suffer a specific disease (e.g., CRC), whereas in another particular disclosure, said control subject is the actual subject under study, which is particularly useful for evaluating the tracking of a disease (e.g., CRC) or for evaluating the efficacy of a treatment for said disease (e.g., CRC), etc., for which the reference level of a given product can be the level of said product determined in a sample from the same subject under study but taken days, weeks, months or even years before for the purpose of evaluating the tracking of the disease, or taken before, for example, the application in the subject of a treatment for said disease for the purpose of evaluating its efficacy.

Due to the variability that can occur among the different subjects in terms of the production of SULF1 protein, the reference level could be obtained from a set of samples from a population of healthy subjects (e.g., subjects who do not suffer CRC) and by calculating the mean level of the product in question (SULF1 protein) in said population of healthy subjects.

The reference level of a certain product, for example, SULF1 protein, can be determined from a reference sample which can be analyzed, for example, simultaneously or consecutively, together with the sample to be analyzed (test sample). The reference level can generally be derived from the normal distribution limits of a physiological amount found in a population of control subjects. Said physiological amount can be determined by several well-known techniques, as described in this description.

According to the present disclosure, said reference level allows discriminating the presence of CRC and can therefore be used in the diagnosis, prognosis or tracking of CRC progression.

The markers and methods provided by the present disclosure are suitable for diagnosing CRC, as well as for predicting the development of a CRC, monitoring the progression of a CRC and/or evaluating the efficacy of treatment administered to a subject who has CRC.

Assays performed by the inventors have clearly shown that the phages identified as MST1, SULF1, NHSL1, SREBF2, GRN and GTF2i allow discriminating between tumor sera (CRC) and control sera by ELISA. Although sensitivity was relatively low for the individual phages, ranging between 46% and 60%, specificity was greater, between 50% and 73.9%, and furthermore the combination of the 6 phages as a predictor of CRC provided an AUC of 0.82 with sensitivity and specificity of 70% and 73.9% [Example 4].

In turn, the combination of SULF1 and MST1 recombinant proteins together with NHSL1, GRN, SREBF2 and GTF2i phages allowed predicting the disease (CRC) with 72% sensitivity and 87% specificity, with an AUC of 0.83. If the age of the patient is further taken into account, the AUC is of 0.91. These markers further allow grouping results by discriminating not only sick individuals, but also different stages of the disease. Likewise, autoantibodies against MST1 and NHSL1 are associated with the clinical prognosis of CRC patients. Therefore, the detection of this panel of autoantibodies in serum is therefore a simple and non-invasive method for the diagnosis/prognosis of CRC.

It has additionally been observed that SULF1 protein overexpression is correlated with the diagnosis of CRC [Figure 3] .

### EXAMPLES

The invention will be illustrated below by means of assays conducted by the inventors, which clearly show the specificity and effectiveness of the method for the diagnosis/prognosis of CRC based on the detection in serum of antibodies against the tumor autoantigens described.

### EXAMPLE 1

### Analysis of sera from CRC patients with microarrays printed with T7 phages

### I. MATERIALS AND METHODS

### CRC and reference sera

The sera used in microarray and survival analyses were obtained from patients at Hospital Universitario de Bellvitge, Instituto Catalán de Oncologia de Barcelona, Hospital Puerta de Hierro de Madrid and Hospital de Cabueñes de Gijón, after obtaining the written consent of all the patients included in the study.

3 sera from CRC patients with Duke's stage B, 3 with stage C and 6 with stage D (3 with liver metastasis and 3 with lung metastasis) were used to select the libraries of CRC-specific T7 phages. 15 sera from CRC patients with different stages, with a mean age of 66.3 years (age range 54-82) and 15 sera from control individuals with a similar mean age and the same sex ratio as the CRC patients were selected for microarray screening.

For the survival analysis, another panel of 95 CRC sera with over 10 years of tracking was tested. The median age was 66.2 years (range between 23-90 years). The clinical data of all the patients are included in Table 3. All the samples were managed anonymously according to the ethical and legal standards of the Consejo Superior de Investigaciones Científicas (CSIC) (Superior Council for Scientific Research).

**Table 3. Clinical-pathological information of patients whose serum was used for the identification and validation of the autoantibodies.**

| CRC patients (n) | 160 | Healthy donors (n) | 61 |
|---|---|---|---|
| Mean age (years) | 67.7 | Mean age (years) | 61. 7 |
| Age range (years) | 23-91 | Age range (years) | 34-89 |

| Sex: | | Sex: | |
|---|---|---|---|
| Male | 65.6% | Male | 60.7% |
| Female | 34.4% | Female | 39.3% |

| Duke's stage: | | | |
|---|---|---|---|
| I | 40.6% | | |
| II | 19.4% | | |
| III | 15.6% | | |
| IV | 24.4% | | |

| Prognosis: | | | |
|---|---|---|---|
| Dead | 33.1% | | |
| Alive | 60% | | |
| Unknown | 6.9% | | |
| Mean survival time (months) | 57.5 | | |

An independent group of sera was used for validation; 50 CRC sera representative of all Duke's stages (A-D), 46 control sera, 10 asymptomatic patients with family history, 2 with hyperplastic polyps, 2 with ulcerative colitis and 43 sera of other types of cancer (bladder, breast, lung, pancreas and stomach).

The sera used were processed in the same manner in the different hospitals; the blood samples were left at room temperature for at least 30 minutes (and a maximum of 60 minutes) to allow clot formation. The samples were subsequently centrifuged at 3000 g at 4°C for 10 minutes. The sera were frozen and stored at -80°C until use.

### Synthesis of T7 phage cDNA library and selection rounds

The total RNA of 3 tumor tissues from CRC patients in Duke's stages A (samples A) and 3 with stage C (samples C), was isolated by means of the reagent Trizol (Invitrogen). Four micrograms of each RNA were used, mixing samples A and samples C separately, for the synthesis of cDNA. The T7 phage cDNA libraries of CRC were constructed using the OrientExpress cDNA synthesis and cloning system (Novagen) according to the manufacturer's instructions. The cDNA was synthesized by means of RT-PCR using the oligonucleotide oligo(dT). The cloning was performed indistinctly in vectors T7Select 415-1 and T7Select 10-3b, which differ in the size of the insert they allow. Phage growth was obtained in *E. coli* strains BL21 and BLT 5403, respectively. The four T7 libraries were titrated making serial dilutions of the T7 phages in Petri dishes. The sizes of the libraries were greater than 10⁶ pfu/mL in all cases. The phage selection rounds were performed using on one hand the combination of the 2 libraries constructed with the T7Select 415-1 vector and on the other the combination of the 2 libraries constructed with the T7Select 10-3b vector.

Negative selection was performed first. To that end, protein A/G coupled magnetic particles (Invitrogen) were incubated with a mixture of 8 control sera (120 µL of mixture of control sera, diluted 1:50, at 4°C overnight) to bind the IgGs of the control subjects. The phages were subsequently incubated with said magnetic particles to remove those phages bound to the IgGs of the control sera. Secondly, 4 mixtures of sera (stage B mixture: from 3 CRC patients with Duke's stage B, stage C mixture: from 3 patients with Duke's stage C, stage D-H mixture: from 3 patients with Duke's stage D and liver metastasis and stage D-P mixture: from 3 patients with Duke's stage D and lung metastasis) were incubated with protein A/G coupled magnetic particles to enrich the phage libraries with CRC specific phages. The phages not retained in the negative selection were incubated with the magnetic particles previously incubated with the different mixtures of sera from CRC patients. The phages bound to said magnetic particles were eluted with 100 µL of 1% SDS and amplified in *E. coli* BLT5406 or BL21. A total of 4 selection rounds were conducted to enrich T7 phage libraries with CRC specific phages. Individual clones of the 8 selections which were printed in nitrocellulose microarrays were finally amplified.

### Phage microarrays

After the amplification of monoclonal phages, the bacteria lysates were centrifuged and the supernatants containing the phages were diluted 1:2 in PBS with 0.1% Tween 20 (PBST) for printing in duplicate in nitrocellulose microarrays (Whatman/Schleicher & Schuell's) with the OmniGrid Spotter robot (GeneMachines, San Carlos, CA). The negative controls used in the printing were BSA (Sigma Aldrich), printing buffer or empty gaps. T7 and human IgG protein (Sigma-Aldrich) were printed as positive controls to verify the quality of the array.

Thirty sera (15 from CRC patients and 15 from normal individuals) were incubated with the phage microarrays as described previously (Chaterjee, M et al. 2006. Cancer Res. 66:1181-1190). Briefly, the slides were equilibrated with PBS at room temperature for 5 minutes and blocked with 3% skim milk in PBS (3% MPBS) for 1 hour at room temperature under stirring. The arrays were subsequently incubated with 6.6 µL of human serum (dilution 1:300), 120 µg of *E. coli* lysate and 0.3 µg of anti T7-tag monoclonal antibody (Novagen) diluted in 2 mL of 3% MPBS for 90 minutes at room temperature. The slides were washed 3 times with PBST for 10 minutes to eliminate nonspecific binding and were incubated with an AlexaFluor 647-conjugated anti-human IgG antibody (Invitrogen) diluted 1:2,000 and an AlexaFluor 555-conjugated anti-mouse IgG antibody (Invitrogen) diluted 1:40,000 in 3% MPBS for detecting the human antibodies bound to T7 phages and the T7 phages, respectively. Subsequently, the microarrays were washed 3 times with PBST, once with PBS and were dried by means of centrifugation at 1200 rpm for 3 minutes. Finally, the slides were scanned in a ScanArrayTM5000 (Packard BioChip Technologies). The Genepix Pro 7 image analysis program (Axon Laboratories) was used to quantify the intensity of the points.

### Statistical analysis

The microarray data were normalized and processed using the Asterias applications (http://asterias.bioinfo.cnio.es/), an interface for using software packages, Limma and marrayNorm from Bioconductor. After applying a background noise correction and global Loess normalization (http://dnmad.bioinfo.cnio.es/), the data were processed to filter the missing values or points with too high of a variance, to combine duplicates and then obtain a single log transformed base 2 value for each phage (http://prep.bioinfo.cnio.es/). The groups of CRC patients and healthy individuals were compared by performing a t-test with the Pomelo II program (http://pomelo2.bioinfo.cnio.es/), where p values were obtained by means of 200000 permutations. The Pomelo II program generated a graph showing the phages with a positive result false discovery rate (FDR) value below 0.15 and an unadjusted p value below 0.05.

The bootstrapping analyses were adjusted by means of a logistic regression model where the probability of being a tumor against the probability of being normal was modeled as a function of the variables (phages and proteins). The age and sex of the patients were also included in the model to correct the possible effects of these variables. The area under the ROC curve (AUC) was calculated to evaluate the predictive ability of the models. The AUC calculated directly with the original model and the complete data set is biased towards high values. Therefore, bootstrapping with 1,000 replicate samples was used to obtain a corrected AUC not biased towards high values, giving an estimate of the AUC that could be obtained with an independent future validation (Efron B. J. Am. Stat Assoc. 1983;78:316-331). The models were adjusted using the Harrell design library (Harrel F. Springer. 2001) with the statistical computing system R (Team RDC, 2009).

### II. RESULTS

The tissue RNA of CRC from 6 patients (three with Duke's stage A and three with Duke's stage C) was used to construct T7 phage libraries containing cDNA fragments in 2 vectors (T7Select 415-1 or T7select 10-3b). After selecting CRC specific phages 8 different libraries enriched in tumor specific phages, depending on the vector and the mixture of sera (B, C, H and P) used during selection, were obtained. A total of 1,536 individual phages were amplified (192 individual phages of each selection) and were printed in duplicate in nitrocellulose arrays. An anti-T7 antibody which allowed observing the presence of a homogenous signal in the array was used as a control of the amount of phage printed in the array. The intensity of the 2 points corresponding to the same phage within the same array and between two different arrays was represented for the purpose of determining intra- and inter- array reproducibility. It was determined that intra- and inter-array reproducibility was good with R2 values of 0.9703 and 0.9091, respectively.

The arrays with 30 sera (15 from patients with different stages of CRC and 15 from healthy controls) were incubated to evaluate the immune response in CRC patients. After quantifying the images and normalizing the data, the signal of the tumor sera was compared with the healthy sera using a t-test with 200,000 permutations. Between the 2 groups 128 phages showed different reactivities, with an FDR < 0.22. Out of those phages, 78 showed increased reactivity in CRC whereas 50 had reduced reactivity in sera with CRC. The representation of the supervised analysis of the 45 phages with the lowest FDR (<0.15) showed a clear separation between CRC patients and healthy individuals.

### EXAMPLE 2

### Identification of the phage-displayed sequences

### I. MATERIALS AND METHODS

Sequencing and analysis of the internal sequences by means of BLASTp

The DNA inserted in the phage genome was amplified by PCR using forward primer T7_up2: 5'-TGCTAAGGACAACGTTATCGG-3' (SEQ ID NO:13) and reverse primer T7_down2: 5'-TTGATACCGGACGTTCAC-3' (SEQ ID NO:14). The PCR products were precipitated with ethanol and sequenced directly with forward primer T7_up2.

A search was conducted in the NCBI database with BLASTp software to find sequence homology for each peptide displayed on the selected phage surface.

### Proteins, antibodies and cell lines

MST1/STK4 and SULF1 human recombinant proteins were expressed in *E. coli.* MST1 cDNA was sub-cloned into pET28a vector (Novagen). SULF1 cDNA was cloned into pDONR221 vector and subsequently into pDEST17 expression vector. The 2 6xHis-MST1 and 6xHis-SULF1 fusion proteins were expressed in *E. coli* strain BL21 (DE3) and purified to homogeneity by means of HisTrap column affinity chromatography (GE Healthcare). Finally, the proteins were dialyzed against PBS and concentrated. EBNA1 protein used as a positive control in ELISA assays was purchased from the company Tebu-Bio.

Antibodies against MST1/STK4, SULF1 and tubulin used in the membrane immunodetection were purchased from the companies Atlas antibodies, Santa Cruz Biotechnology and Sigma, respectively. The TrueBlot peroxidase-conjugated anti-rabbit IgG antibody was purchased from the company eBioscence and the peroxidase-conjugated anti-mouse IgG and anti-human IgG antibodies were purchased from DakoCytomation.

Colorectal cancer cell lines RKO, Caco2, Hct15, Hct116, Colo320, SW480, SW48, KM12C, KM12SM, HT29, Colo205 and reference cell lines (HEK293 and MOLT4) were grown according to protocols established. Peripheral blood lymphocytes (PBL) and monocytes were isolated from a healthy donor.

### Western-blot analysis

The preparation of the cell lines and paired tissue extracts was performed according to the following protocol. The cells and the tissues were washed twice with PBS before lysis with 500 µL of 0.5% SDS with protease inhibitors (Roche Applied Science). The concentration of the extracts was determined by means of the 2D-Quant kit (GE Healthcare) after clarifying the sample by means of centrifugation at 12,000 g for 15 minutes at 4°C.

25 µg of protein extract were separated in 10% SDS-PAGE gel and transferred to nitrocellulose membranes (Hybond-C Extra) according to established protocols (Babel et al. Mol. Cell Proteomics 2009; 8:2382-95). The membrane was blocked with 3% MPBS and incubated overnight at 4°C with the antibodies against MST1 (1:1,000 dilution), SULF1 (1:3,000 dilution) or tubulin (1:5,000 dilution). Immunodetection was performed using an HRP-conjugated anti-mouse IgG antibody (1:5,000 dilution) or an HRP-conjugated anti-rabbit IgG antibody (1:5,000 dilution). Antibody binding was finally detected using ECL (GE Healthcare) or SuperSignal Femto (Pierce).

### ELISA

T7 phage capture ELISA plates (Novagen) were blocked for 2 hours at 37°C with 3% MPBS and incubated overnight with 100 µL of the bacterial lysate of the phages diluted in 3% MPBS. After washing 3 times with PBST, the plates were blocked with 3% MPBS for 1 hour at 37°C and incubated with 100 µL of human serum (1:50 dilution in 3% MPBS) for 1 hour at 37°C. After 3 additional washes, peroxidase-labeled anti-human IgG antibody (1:3,000 in 3% MPBS) was added for 2 hours at room temperature. The signal was detected with 3,3',5,5'-tetramethylbenzidine substrate (Sigma) for 10 minutes, stopping the reaction with 1 M HCl and measuring the signal at 450 nm.

The competitive assay between the peptides displayed on phage surfaces and the recombinant proteins was performed using the T7 phage capture plates (Novagen) following the preceding protocol, except the human sera were pre-incubated overnight at 4°C with serial dilutions of MST1, SULF1 or GST proteins. The sera thus pre-incubated were tested against EBNA1 in ELISA plates (Maxisorp, Nunc) as a positive control to verify that the competition for IgGs between the phage and its respective recombinant protein was specific.

The ELISAs with MST1, SULF1 and EBNA1 proteins were performed as described previously (Babel et al. Mol. Cell Proteomics 2009; 8:2382-95). The concentration of CEA in the sera from CRC patients and the control sera was determined by means of a specific immunological test following manufacturer's recommendations (MP Biomedicals).

### Statistical analysis

The ELISA data for each individual marker (full length phage or protein) were evaluated calculating an ROC curve (receiver operating characteristic curve). The corresponding area under the curve (AUC) was calculated using the JMP7 program (SAS). The mean and standard deviation of the immunohistochemistry results were calculated using the Microsoft Office Excel 2007 program. The one-tailed Student's t-test was carried out using the immunohistochemistry results, assuming that the unequal variances for determining the means of the normal and tumor groups were significantly different from one another.

### II. RESULTS

Forty-three unique amino acid sequences were obtained fused to T7 phage capsid protein 10B among the 78 phages showing increased reactivity in sera from CRC patients.

Out of the 43 unique phages those phages which contained between 8 and 20 residues with high homology to known protein sequences, which appeared a greater number of times with the same amino acid sequence and which had a low p value, were selected to verify the results. Sequences homologous to MST1/STK4, SULF1, NHSL1, SREBF2, GRN and GTF2i proteins were identified. All of them had a significantly greater signal in the microarray with the serum from CRC patients than with the control sera (Figure 1). The phages were identified by the name of the protein with which homologous sequences were identified. MST1/STK4 protein was identified previously as a tumor-associated antigen in CRC using commercial protein microarrays (Babel et al. Mol. Cell Proteomics 2009;8:2382-95) and the SULF1 gene was described as being overexpressed in a transcriptome analysis of CRC (Madoz-Gurpide et al. Mol Cell Proteomics, 2006;5:1471-83).

A competitive IgG assay was performed between the phages and SULF1 and MST1 human recombinant proteins for the purpose of confirming that the phage-displayed peptides the homologous sequence of which belonged to SULF1 and MST1 proteins. The binding of the immunoglobulins present in the human sera to the 2 phages was inhibited in a dose-dependent manner with MST1 and SULF1 recombinant proteins (Figure 2A). GST did not affect the binding of IgGs to phages (negative control). As a specific inhibition control, it was observed that the binding of the antibodies of the patients to EBNA protein was not affected by incubation with MST1 or SULF1 proteins.

In addition, it was determined that the sequences of the phage-displayed peptides were located in the C-terminal region of MST1 and in the N-terminal region of SULF1 (Figure 2B).

All these results confirm that the displayed peptides correspond to immunodominant epitopes of MST1 and SULF1 proteins.

### EXAMPLE 3

### The identified proteins are overexpressed in colorectal cancer

Tumor-associated antigens recognized by autoantibodies are generally overexpressed in cell lines and in tumor tissues. Meta analysis of mRNA expression levels of the homologous proteins corresponding to the 6 phages selected [MST1/STK4, SULF1, NHSL1, SREBF2, GRN and GTF2i] was performed with the Oncomine microarray database (Rhodes et al. Neoplasia 2004;6:1-6) (Figure 3A). It was found that SULF1 was the most overexpressed gene in colon cancer, followed by GTF2i, MST1, GRN, NHSL1 and SREBF2. In addition, membrane immunodetection was performed with the antibodies against MST1 and SULF1 using 11 CRC cell lines and tumor tissues from CRC patients representing the different stages of disease progression (Figure 3B). It was found that MST1 and SULF1 proteins were expressed in most colon cancer cell lines. The greater expression of SULF1 was observed in metastatic cell lines (SW48, HT29 and COLO205) and in CRC tumor tissue in late stages.

The cellular expression patterns of the selected proteins were characterized by means of immunohistochemistry (TMA) using independent CRC tumors arranged in microarrays or by means of meta analysis of tissue microarray data obtained from the Human Protein Atlas (Berglund et al. Mol Cell Proteomics. 2008;7:2019-27) (Figure 3C). In all cases, more abundant expression of the protein studied was detected in tumor tissues.

Therefore, there is good correlation between the presence of autoantibodies, the abundance of proteins and gene expression.

### EXAMPLE 4

### Validation of the predictor formed by phage-displayed peptides and their homologous proteins

An independent set of 96 serum samples (50 with colorectal cancer with 19 samples in early stages (A+B) and 46 healthy controls) were used for the validation of the results. MST1, SULF1, NHSL1, SREBF2, GRN and GTF2i phages were tested for their ability to discriminate between tumor sera and control sera by ELISA. ROC curves were constructed for each of the markers with the ELISA results. While sensitivity was relatively low for individual phages, ranging between 46% and 60%, specificity was higher, between 50% and 73.9%. The data was fitted to a logistic curve performing linear regressions and producing different models with different combinations of phages to investigate if different combinations of phages showed greater precision in discriminating healthy individuals from cancer patients. Therefore, the result of the combination of the 6 phages as a predictor of CRC gives an AUC of 0.82 with sensitivity and specificity of 70% and 73.9%, respectively (Table 4).

**Table 4. Data from the ROC curves obtained from the ELISA values of the validation of both individual phages and of combined phages.**

| Phage-displayed peptide | Specificity (%) | Sensitivity (%) | AUC |
|---|---|---|---|
| SULF1 | 73.9 | 50.0 | 0.63 |
| NHSL1 | 50.0 | 56.0 | 0.59 |
| MST1 | 71.7 | 46.0 | 0.58 |
| GTF2i | 52.2 | 60.0 | 0.57 |
| SREBF2 | 69.6 | 54.0 | 0.61 |
| GRN | 50.0 | 58.0 | 0.53 |
| Combination of 6 phages | 73.9 | 70.0 | 0.82 |

The following step consisted of seeing if the replacement of the phages with their MST1 and SULF1 recombinant proteins would improve the discriminatory power of the model (Figure 4). The results confirmed a significant improvement of the prediction using the recombinant proteins, with AUCs of 0.71 and 0.74 for SULF1 and MST1 proteins against 0.63 and 0.58 of the respective phages (Table 2). By combining the two proteins (SULF1 and MST1) and the four phages (NHSL1, SREBF2, GRN and GTF2i), the AUC increased to 0.86 with sensitivity of 82.6% and specificity of 70% (Figure 6A). The CEA values were lower (AUC 0.81) and when combined with the remaining predictions they barely improved the model (AUC 0.89). Different AUC estimations were further performed in the validation step to compare not only CRC versus healthy, but also CRC versus reference sera and healthy versus other tumors (Figure 6). The most relevant result was the capacity of the model to discriminate not only CRC from healthy sera (AUC 0.86) (Figure 6A), but also CRC from all the reference sera, which included other colon-related pathologies (AUC 0.85) (Figure 6B). Notably, the panel seemed to not suitably discriminate healthy controls from other tumors (AUC 0.63) (Figure 6C). The panel further seemed to significantly discriminate healthy controls from asymptomatic patients with a family history of CRC (AUC 0.78). Bootstrapping analysis

Bootstrapping was also performed to obtain the corrected AUC. The initial model included linear terms for all the phages and proteins, together with two other variables: sex and age of the patients. The corrected AUC value was 0.83 with this model.

This model was probably more complex than necessary. For that reason, a variable selection was performed with the Akaike information criterion as the endpoint. The final model only retained 3 proteins (GRN, MST1 and SULF1), in addition to the age of the patients (Table 5). However, to prevent an overestimate of the predictive capacity of the model, estimated corrected AUC values were obtained by means of bootstrapping the entire variable selection process (i.e., the complete model with 8 variables was performed and the Akaike information criterion was used for each bootstrap sample). The corrected AUC was 0.84. Bootstrapping also provided information on selection process stability; most bootstrapping models contained four, five, six or seven variables. Some of the variables appeared in most of the models; the GRN phage in 976, protein SULF1 in 954, age in 952 and MST1 protein in 833.

This model was further used for predicting the probability of being CRC from a group of 57 sera that comprised various pathologies. A dot-plot (Figure 6D) was generated, showing the individual probability for each subject. Great variability in probability was observed within each group, but the median was way below 0.5, indicating a low probability of having CRC.

**Table 5. Final model with bootstrapping after the selection model**

| | Estimate | Std Error. | z value | Pr(>\|z\|) |
|---|---|---|---|---|
| (Intercept*) | -5.92318 | 2.15625 | -2.747 | 0.00601** |
| GRN-phage | -6.28345 | 2.91418 | -2.156 | 0.03107* |
| NHSL1- phage | 6.40976 | 2.8367 | 2.26 | 0.02385* |
| GTF2B- phage | -9.00788 | 2.63827 | -3.414 | 0.00064*** |
| SREBF2-phage | 10.31184 | 3.30888 | 3.116 | 0.00183** |
| MST1- phage | 3.53576 | 1.32002 | 2.679 | 0.00739** |
| SULF1- phage | 7.26445 | 2.97056 | 2.445 | 0.01447* |
| Sex | -0.98593 | 0.65076 | -1.515 | 0.12976 |
| Age | 0.05674 | 0.0232 | 2.446 | 0.01444* |

| | | | | |
|---|---|---|---|---|
| **The intercept is the log value (p*/*(1-p)), where p is the probability of being a tumor, when the value of the other variables in the model is 0. Estimate, estimated coefficient (slope); Std. Error, standard error of the specified variable; z value or Wald statistic, which is exactly equal to the estimated coefficient divided by its standard error; Pr(>*\|*z*\|*), p value of the Wald test for that specific coefficient comparing the z value to the normal standard; *, degree of significance.* | | | | |

The predictor was subsequently tested according to the stage of the patient, using the model with 6 markers (4 phages + 2 proteins) plus the age of the patients. The AUC corrected using bootstrapping was 0.786 for stages A+B; 0.857 for stage C; and 0.849 for stage D. If the same test with CEA values is applied, the corrected AUC values were 0.742 for stages A+B, 0.770 for stage C and 0.973 for stage D. These results indicate clear superiority of the predictive model for the diagnosis of CRC in stages A, B and C, CEA being better for stage D, as was expected.

### Comparative EXAMPLE 5 Autoantibodies against MST1 and NHSL1 are associated with the clinical prognosis of CRC patients

After having demonstrated that the predictive panel was capable of identifying patients with tumors in both early and late stages, the potential prognosis of autoantibodies was investigated by analyzing their association with absolute patient survival.

Survival was estimated by means of standard Kaplan-Meier method. The prognostic value of candidate antibodies was evaluated by means of stage-stratified Cox's proportional hazards models. The p values were obtained from the probability ratio test. Only the antibodies found as being significant for diagnosis were evaluated for prognosis in a step-by-step multivariate process.

Patients with low levels of antibodies against MST1 showed a lower cumulative survival than those with high levels of autoantibodies (p=0.08). In contrast, patients with higher titers of antibodies against NHSL1 were associated with lower survival (p=0.06). The combination of both effects improves the statistical value of the prediction (0.032) (Figure 5) and confirms the survival prediction. Collectively, these data indicate a correlation between the presence of antibodies against MST1 and NHSL1 with the clinical prognosis of colorectal cancer patients.

This prognostic value was most important for early stages of the disease with a risk index of 5.1; though it was not significant (p=0.12) because only 8 events were observed in the 47 patients in stages I-II. A similar effect was further observed in the analysis of disease-free survival when the patients were restricted to tumor-free surgical resection margins.

## Claims

1. A method for detecting an autoantibody in a sample, comprising:
a) contacting a sample from a subject with an antibody capturing entity (ACE), wherein said ACE is an ACE comprising the amino acid sequence shown in SEQ ID NO: 4 or a peptide or protein having at least 90% sequence identity with respect to SEQ ID NO: 4 and that is recognized by an autoantibody against SEQ ID NO: 4;
and
b) detecting the formation of a complex between an autoantibody and said ACE,
wherein the detection of said autoantibody-ACE complex is indicative of the presence of said autoantibody in said sample, wherein the sample is blood, plasma or serum.

2. A method according to claim 1, wherein the sample is a sample from a subject suspected of having colorectal cancer (CRC).

3. The method according to claim 1 or 2, comprising the detection of an autoantibody against an ACE comprising the amino acid sequence shown in SEQ ID NO: 4 or a protein or peptide having at least 90% sequence identity with respect to SEQ ID NO: 4 containing an epitope recognizable by an autoantibody against SEQ ID NO: 4, and furthermore the detection of an autoantibody selected from the group consisting of:
i) an autoantibody against an ACE comprising the amino acid sequence shown in SEQ ID NO: 2 or a protein or peptide having at least 90% sequence identity with respect to SEQ ID NO: 2 containing an epitope recognizable by an autoantibody against SEQ ID NO: 2;
ii) an autoantibody against an ACE comprising the amino acid sequence shown in SEQ ID NO: 6 or a protein or peptide having at least 90% sequence identity with respect to SEQ ID NO: 6 thereof containing an epitope recognizable by an autoantibody against SEQ ID NO: 6;
iii) an autoantibody against an ACE comprising the amino acid sequence shown in SEQ ID NO: 3 or a protein or peptide having at least 90% sequence identity with respect to SEQ ID NO: 3 thereof containing an epitope recognizable by an autoantibody against SEQ ID NO: 3;
iv) an autoantibody against an ACE comprising the amino acid sequence shown in SEQ ID NO: 5 or a protein or peptide having at least 90% sequence identity with respect to SEQ ID NO: 5 thereof containing an epitope recognizable by an autoantibody against SEQ ID NO: 5;
v) an autoantibody against an ACE comprising the amino acid sequence shown in SEQ ID NO: 1 or a protein or peptide having at least 90% sequence identity with respect to SEQ ID NO: 1 thereof containing an epitope recognizable by an autoantibody against SEQ ID NO: 1; and
vi) any combination of autoantibodies (i) to (v).

4. The method according to claim 1 or 2, wherein said ACE is sulfatase 1 (SULF1) or a protein or peptide having at least 90% sequence identity with respect to SULF1 containing an epitope recognizable by an autoantibody against SULF1,
or alternatively
wherein said ACE is a phage comprising the amino acid sequence shown in SEQ ID NO: 4 or a protein or peptide having at least 90% sequence identity with respect to SEQ ID NO: 4 containing an epitope recognizable by an autoantibody against SEQ ID NO: 4, wherein said amino acid sequence is exposed on the phage surface.

5. The method according to claim 2, further comprising correlating the formation of the autoantibody-ACE complex in the sample from the subject with a diagnosis of CRC.

6. The method according to claim 2, further comprising comparing the immunoreactivity of the sample with the immunoreactivity of a second sample from the same subject in a later time period.

7. The method according to claim 6, wherein said second sample from the subject has been obtained after said subject has been treated for CRC.

8. An antibody capturing entity (ACE) comprising the amino acid sequence shown in SEQ ID NO: 4, wherein said ACE is not sulfatase-1 (SULF1) protein.

9. The antibody capturing entity (ACE) according to claim 8, wherein said ACE is a phage comprising the amino acid sequence shown in SEQ ID NO: 4, wherein said amino acid sequence is exposed on the phage surface.

10. A composition comprising an antibody capturing entity (ACE) according to claim 8.

11. The composition according to claim 10, comprising at least one ACE according to claim 8, and at least one protein selected from the group consisting of sulfatase 1 (SULF1) protein or a protein or peptide having at least 90% sequence identity with respect to SULF1 and recognizable by an autoantibody against SULF1, MST1 protein or a protein or peptide having at least 90% sequence identity with respect to MST1 and recognizable by an autoantibody against MST1, and their combinations.

12. A composition comprising sulfatase 1(SULF1) protein or a protein or peptide having at least 90% sequence identity with respect to SULF1 and recognizable by an autoantibody against SULF1 and MST1 protein or a protein or peptide having at least 90% sequence identity with respect to MST1 and recognizable by an autoantibody against MST1 and an antibody capturing entity (ACE) according to claim 8.

13. A kit comprising a composition according to any of claims 10 to 12.

14. An in vitro use of a kit according to claim 13 for detecting an antibody in a sample, or for detecting an autoantibody in a subject suspected of having colorectal cancer (CRC), or for diagnosing whether a subject has CRC, or for determining the risk of a subject developing CRC, or for monitoring CRC progression in a subject, or for evaluating the efficacy of a treatment against CRC, or for predicting survival of a subject who has CRC.

## Patentansprüche

1. Ein Verfahren zum Nachweis eines Autoantikörpers in einer Probe, umfassend:
a) Kontaktieren einer Probe eines Individuums mit einer Antikörper-Erfassungseinheit (ACE), wobei die Antikörper-Erfassungseinheit eine Antikörper-Erfassungseinheit ist, die die Aminosäuresequenz umfasst, die in SEQ ID Nr.: 4 gezeigte ist, oder ein Peptid oder Protein mit mindestens 90 % Sequenzidentität in Bezug auf SEQ ID Nr.: 4, und das von einem Autoantikörper gegen SEQ ID Nr.: 4 erkannt wird; und
b) Nachweisen der Bildung eines Komplexes zwischen einem Autoantikörper und der Antikörper-Erfassungseinheit,
wobei der Nachweis des Autoantikörper-Antikörper-Erfassungseinheit-Komplexes indikativ für das Vorhandensein des Autoantikörpers in der Probe ist, wobei die Probe Blut, Plasma oder Serum ist.

2. Das Verfahren nach Anspruch 1, wobei die Probe eine Probe von einem Individuum ist, bei dem der Verdacht auf Darmkrebs (CRC) besteht.

3. Das Verfahren nach Anspruch 1 oder 2, umfassend den Nachweis eines Autoantikörpers gegen eine Antikörper-Erfassungseinheit, die eine Aminosäuresequenz umfasst, die in SEQ ID Nr.: 4 gezeigt ist, oder ein Protein oder Peptid mit mindestens 90% Sequenzidentität in Bezug auf SEQ ID Nr.: 4, das ein Epitop beinhaltet, das für einen Autoantikörper gegen SEQ ID Nr.: 4 erkennbar ist, und außerdem den Nachweis eines Autoantikörpers, ausgewählt aus der Gruppe bestehend aus:
i) einem Autoantikörper gegen eine Antikörper-Erfassungseinheit, der die Aminosäuresequenz umfasst, die in SEQ ID Nr.: 2 gezeigt ist, oder einem Protein oder Peptid mit mindestens 90% Sequenzidentität in Bezug auf SEQ ID Nr.: 2, das ein Epitop beinhaltet, dass für einen Autoantikörper gegen SEQ ID Nr.: 2 erkennbar ist;
ii) einem Autoantikörper gegen eine Antikörper-Erfassungseinheit, der die Aminosäuresequenz umfasst, die in SEQ ID Nr.: 6 gezeigt ist, oder einem Protein oder Peptid mit mindestens 90% Sequenzidentität in Bezug auf SEQ ID Nr.: 6, das ein Epitop beinhaltet, das für einen Autoantikörper gegen SEQ ID Nr.: 6 erkennbar ist;
iii) einem Autoantikörper gegen eine Antikörper-Erfassungseinheit, der die Aminosäuresequenz umfasst, die in SEQ ID Nr.: 3 gezeigt ist, oder einem Protein oder Peptid mit mindestens 90% Sequenzidentität in Bezug auf SEQ ID Nr.: 3, das ein Epitop beinhaltet, das für einen Autoantikörper gegen SEQ ID Nr.: 3 erkennbar ist;
iv) einem Autoantikörper gegen eine Antikörper-Erfassungseinheit, der die Aminosäuresequenz umfasst, die in SEQ ID Nr.: 5 gezeigt ist, oder einem Protein oder Peptid mit mindestens 90% Sequenzidentität in Bezug auf SEQ ID Nr.: 5, das ein Epitop beinhaltet, das für einen Autoantikörper gegen SEQ ID Nr.: 5 erkennbar ist;
v) einem Autoantikörper gegen eine Antikörper-Erfassungseinheit, der die Aminosäuresequenz umfasst, die in SEQ ID Nr.: 1 gezeigt ist, oder einem Protein oder Peptid mit mindestens 90% Sequenzidentität in Bezug auf SEQ ID Nr.: 1, das ein Epitop beinhaltet, das für einen Autoantikörper gegen SEQ ID Nr.: 1 erkennbar ist;
vi) und jede Kombination der Autoantikörper (i) bis (v).

4. Das Verfahren nach Anspruch 1 oder 2, wobei die Antikörper-Erfassungseinheit Sulfatase 1 (SULF1) oder ein Protein oder Peptid mit mindestens 90% Sequenzidentität in Bezug auf SULF1 ist, das ein Epitop beinhaltet, das von einem Autoantikörper gegen SULF1 erkannt wird,
oder alternativ
wobei die Antikörper-Erfassungseinheit ein Phage ist, der die Aminosäuresequenz umfasst, die in SEQ ID Nr.: 4 gezeigt ist, oder ein Protein oder Peptid mit mindestens 90% Sequenzidentität in Bezug auf SEQ ID Nr.: 4, das ein Epitop beinhaltet, das für einen Autoantikörper gegen SEQ ID Nr.: 4 erkennbar ist, wobei die Aminosäuresequenz auf der Phagenoberfläche präsentiert wird.

5. Das Verfahren nach Anspruch 2, das ferner Korrelieren der Bildung des Autoantikörper-ACE-Komplexes in einer Probe eines Probanden, mit einer Darmkrebs Diagnose, umfasst.

6. Das Verfahren nach Anspruch 2, das ferner Vergleichen der Immunreaktivität einer Probe mit der Immunreaktivität einer zweiten Probe des gleichen Probanden zu einem späteren Zeitpunkt umfasst.

7. Das Verfahren nach Anspruch 6, wobei die zweite Probe des Individuums erhalten wurde, nachdem das Individuum gegen Darmkrebs behandelt wurde.

8. Eine Antikörper-Erfassungseinheit (ACE), die die Aminosäuresequenz umfasst, die in SEQ ID Nr.: 4 gezeigt ist, wobei die Antikörper-Erfassungseinheit kein Sulfatase-1 (SULF1) Protein ist.

9. Die Antikörper-Erfassungseinheit (ACE) nach Anspruch 8, wobei die ACE ein Phage ist, der die Aminosäuresequenz umfasst, die in SEQ ID Nr.: 4 gezeigt ist, wobei die Aminosäuresequenz auf der Phagenoberfläche präsentiert wird.

10. Eine Zusammensetzung, die eine Antikörper-Erfassungseinheit (ACE) nach Anspruch 8 umfasst.

11. Die Zusammensetzung nach Anspruch 10, die mindestens eine ACE nach Anspruch 8 und mindestens ein Protein umfasst, ausgewählt aus der Gruppe bestehend aus Sulfatase 1 (SULF1) Protein oder einem Protein oder Peptid mit mindestens 90% Sequenzidentität in Bezug auf SULF1, und das für einen Autoantikörper gegen SULF1 erkennbar ist, MST1 Protein oder einem Protein oder Peptid mit mindestens 90 % Sequenzidentität in Bezug auf MST1, und das für einen Autoantikörper gegen MST1 erkennbar ist, und ihre Kombinationen.

12. Eine Zusammensetzung, umfassend ein Sulfatase-I (SULF1) Protein oder ein Protein oder Peptid mit mindestens 90% Sequenzidentität in Bezug auf SULF1, und das für einen Autoantikörper gegen SULF1- und MST1-Protein erkennbar ist, oder ein Protein oder Peptid mit mindestens 90% Sequenzidentität in Bezug auf MST1, und das für einen Autoantikörper gegen MST1 erkennbar ist, und eine Antikörper-Erfassungseinheit (ACE) nach Anspruch 8.

13. Ein Kit, das eine Zusammensetzung nach einem der Ansprüche 10 bis 12 umfasst.

14. Eine *in vitro* Verwendung eines Kits nach Anspruch 13 zum Nachweis eines Antikörpers in einer Probe oder zum Nachweis eines Autoantikörpers bei einem Individuum, bei dem der Verdacht besteht, dass es Darmkrebs (CRC) hat, oder zur Diagnose, ob ein Individuum Darmkrebs hat, oder zur Bestimmung des Risikos, dass ein Individuum Darmkrebs entwickelt, oder für die Überwachung des Krankheitsverlaufs in einem Individuum mit Darmkrebs oder für die Bewertung der Wirksamkeit einer Behandlung gegen Darmkrebs, oder für die Vorhersage des Überlebens eines Individuums der Darmkrebs hat.

## Revendications

1. Une méthode pour détecter un autoanticorps dans un échantillon, comprenant :
a) le fait de mettre en contact un échantillon provenant d'un sujet avec une entité de capture d'anticorps (ACE), ladite ACE étant une ACE comprenant la séquence d'acides aminés représentée dans SEQ ID NO: 4 ou un peptide ou une protéine ayant au moins 90 % d'identité de séquence par rapport à SEQ ID NO: 4 et qui est reconnue par un autoanticorps contre SEQ ID NO: 4 ; et
b) le fait de détecter la formation d'un complexe entre un autoanticorps et ladite ACE,
la détection dudit complexe autoanticorps-ACE étant indicative de la présence dudit autoanticorps dans ledit échantillon, l'échantillon étant du sang, du plasma ou du sérum.

2. Une méthode selon la revendication 1, dans laquelle l'échantillon est un échantillon provenant d'un sujet suspecté d'avoir un cancer colorectal (CRC).

3. La méthode selon la revendication 1 ou la revendication 2, comprenant la détection d'un autoanticorps contre une ACE comprenant la séquence d'acides aminés présentée dans SEQ ID NO: 4 ou une protéine ou un peptide ayant au moins 90% d'identité de séquence par rapport à SEQ ID NO: 4 contenant un épitope reconnaissable par un autoanticorps contre SEQ ID NO: 4, et en outre la détection d'un autoanticorps choisi dans le groupe constitué par :
i) un autoanticorps contre une ACE comprenant la séquence d'acides aminés présentée dans SEQ ID NO: 2 ou une protéine ou un peptide ayant au moins 90 % d'identité de séquence par rapport à SEQ ID NO: 2 contenant un épitope reconnaissable par un autoanticorps contre SEQ ID NO: 2 ;
ii) un autoanticorps contre une ACE comprenant la séquence d'acides aminés présentée dans SEQ ID NO: 6 ou une protéine ou un peptide ayant au moins 90 % d'identité de séquence par rapport à SEQ ID NO: 6 de celui-ci contenant un épitope reconnaissable par un autoanticorps contre a SEQ ID NO: 6 ;
iii) un autoanticorps contre une ACE comprenant la séquence d'acides aminés présentée dans SEQ ID NO: 3 ou une protéine ou un peptide ayant au moins 90 % d'identité de séquence par rapport à SEQ ID NO: 3 de celui-ci contenant un épitope reconnaissable par un autoanticorps contre SEQ ID NO: 3 ;
iv) un autoanticorps contre une ACE comprenant la séquence d'acides aminés présentée dans SEQ ID NO: 5 ou une protéine ou un peptide ayant au moins 90 % d'identité de séquence par rapport à SEQ ID NO: 5 de celui-ci contenant un épitope reconnaissable par un autoanticorps contre SEQ ID NO: 5 ;
v) un autoanticorps contre une ACE comprenant la séquence d'acides aminés présentée dans SEQ ID NO: 1 ou une protéine ou un peptide ayant au moins 90% d'identité de séquence par rapport à SEQ ID NO: 1 de celui-ci contenant un épitope reconnaissable par un autoanticorps contre SEQ ID NO: 1 ; et
vi) toute combinaison d'autoanticorps (i) à (v).

4. La méthode selon la revendication 1 ou la revendication 2, dans laquelle ladite ACE est la sulfatase 1 (SULF1) ou une protéine ou un peptide ayant une identité de séquence d'au moins 90 % par rapport à SULF1 contenant un épitope reconnaissable par un autoanticorps contre SULF1,
ou alternativement
dans laquelle ladite ACE est un phage comprenant la séquence d'acides aminés présentée dans SEQ ID NO : 4 ou une protéine ou un peptide ayant au moins 90 % d'identité de séquence par rapport à SEQ ID NO : 4 contenant un épitope reconnaissable par un autoanticorps contre SEQ ID NO : 4, ladite séquence d'acides aminés étant exposée sur la surface du phage.

5. La méthode selon la revendication 2, comprenant en outre le fait de corréler la formation du complexe autoanticorps-ACE dans l'échantillon provenant du sujet avec un diagnostic de CRC.

6. La méthode selon la revendication 2, comprenant en outre le fait de comparer l'immuno-réactivité de l'échantillon avec l'immuno-réactivité d'un deuxième échantillon provenant du même sujet dans une période de temps ultérieure.

7. La méthode selon la revendication 6, dans laquelle ledit deuxième échantillon provenant du sujet a été obtenu après que ledit sujet ait été traité pour un CRC.

8. Une entité de capture d'anticorps (ACE) comprenant la séquence d'acides aminés présentée dans SEQ ID NO: 4, ladite ACE n'étant pas la protéine sulfatase-1 (SULF1).

9. L'entité de capture d'anticorps (ACE) selon la revendication 8, dans laquelle ladite ACE est un phage comprenant la séquence d'acides aminés présentée dans SEQ ID NO: 4, ladite séquence d'acides aminés étant exposée sur la surface du phage.

10. Une composition comprenant une entité de capture d'anticorps (ACE) selon la revendication 8.

11. La composition selon la revendication 10, comprenant au moins une ACE selon la revendication 8, et au moins une protéine choisie dans le groupe constitué par la protéine sulfatase 1 (SULF1) ou une protéine ou un peptide ayant au moins 90 % d'identité de séquence par rapport à SULF1 et reconnaissable par un autoanticorps contre SULF1, la protéine MST1 ou une protéine ou un peptide ayant au moins 90 % d'identité de séquence par rapport à MST1 et reconnaissable par un autoanticorps contre MST1, et leurs combinaisons.

12. Une composition comprenant la protéine sulfatase 1(SULF1) ou une protéine ou un peptide ayant au moins 90 % d'identité de séquence par rapport à SULF1 et reconnaissable par un autoanticorps contre SULF1 et une protéine MST1 ou une protéine ou un peptide ayant au moins 90 % d'identité de séquence par rapport à MST1 et reconnaissable par un autoanticorps contre MST1 et une entité de capture d'anticorps (ACE) selon la revendication 8.

13. Un kit comprenant une composition selon l'une des revendications 10 à 12.

14. Une utilisation in vitro d'un kit selon la revendication 13 pour détecter un anticorps dans un échantillon, ou pour détecter un autoanticorps chez un sujet suspecté d'avoir un cancer colorectal (CRC), ou pour diagnostiquer si un sujet a un CRC, ou pour déterminer le risque qu'un sujet développe un CRC, ou pour surveiller la progression du CRC chez un sujet, ou pour évaluer l'efficacité d'un traitement contre le CRC, ou pour prédire la survie d'un sujet qui a un CRC.
